(19) Europäisches Patentamt
European Patent Office
Office européen des brevets

(11) Veröffentlichungsnummer: **0 271 443 B1**

(12) # EUROPÄISCHE PATENTSCHRIFT

(45) Veröffentlichungstag der Patentschrift: **17.10.90**

(51) Int. Cl.⁵: **C07C 275/06**, A61K 31/21

(21) Anmeldenummer: **87810714.3**

(22) Anmeldetag: **03.12.87**

(54) **N,N'-Disubstituierte Harnstoffe und Verfahren zu ihrer Herstellung.**

(30) Priorität: **09.12.86 CH 4907/86**
**23.07.87 CH 2793/87**

(43) Veröffentlichungstag der Anmeldung:
**15.06.88 Patentblatt 88/24**

(45) Bekanntmachung des Hinweises auf die Patenterteilung:
**17.10.90 Patentblatt 90/42**

(84) Benannte Vertragsstaaten:
**AT BE CH DE ES FR GB GR IT LI LU NL SE**

(56) Entgegenhaltungen:
**Chemical Abstracts, Vol. 100, Nr. 23, 4. Juni 1984, Columbus, Ohio, USA Hershko, Chaim et al. "Phenolic ethylendiamine derivatives; a study of orally effective iron chelators"**
**Helvetica Chimica Acta, vol. XLVI, part II, fasciculus IV-V, edition Helvetica Chimica Acta, Basel 1963 H.Bickel et al. "Zur Kenntnis von Desferrioxamin B" Seiten 1097-1832**

(73) Patentinhaber: **CIBA-GEIGY AG, Klybeckstrasse 141, CH-4002 Basel(CH)**

(72) Erfinder: **Peter, Heinrich, Dr., Bündtenweg 69, CH-4102 Binningen(CH)**

EP 0 271 443 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue N,N'-disubstituierte Harnstoffe, abgeleitet von Hydroxamsäure, insbesondere von Trihydroxamsäuren, welche unter der Bezeichnung Ferrioxamine und speziell Desferrioxamine als Stoffwechselprodukt von Mikroorgansimen, insbesondere Actinomyceten, bekannt sind. Darunter betrifft die Erfindung vor allem die von Desferrioxamin B abgeleiteten N,N'-disubstituierten Harnstoffe und deren O-Acylate der allgemeinen Formel I,

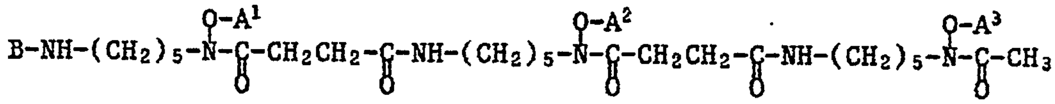

(I)

in welcher B für einen Carbamoylrest der Teilformel -CO-NH-Alk-CO-O-$R^1_a$ (II) steht, worin $R^1_a$ $C_1$-$C_4$-Alkyl oder $C_2$-$C_4$-Alkenyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und jedes der Symbole $A^1$, $A^2$ und $A^3$ unabhängig von den anderen für Wasserstoff, einen von einer Carbonsäure abgeleiteten Acylrest Ac oder einen oben definierten Carbamoylrest der Teilformel II steht, sowie Salze von solchen Verbindungen mit salzbildenden Eigenschaften.

Die Erfindung betrifft auch Verfahren zur Herstellung der oben genannten Verbindungen, sowie diese Verbindungen enthaltende pharmazeutische Zusammensetzungen und Verfahren zu ihrer Her stellung; ferner auch die therapeutische Anwendung dieser Verbindungen und diese enthaltender pharmazeutischer Zusammensetzungen bei Warmblütern einschliesslich des Menschen.

Desferrioxamin B, der Grundstoff der Acylate der vorliegenden Erfindung, ist bereits längere Zeit bekannt (H. Bickel, H. Keberle und E. Vischer: Helv.Chim.Acta <u>46</u>, 1385-9 [1963]). Seine chemische Struktur entspricht der Formel

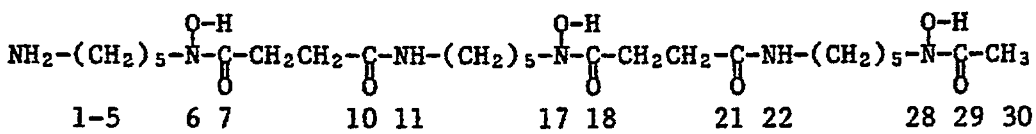 (A)

und wird im Einklang mit der Regel C-06 (Austausch-Nomenklatur der offiziellen IUPAC-Nomenklatur) mit dem systematischen Namen 6,17,28-Trihydroxy-7,10,18,21,29-pentaoxo-6,11,17,22,28-pentaazatriacontylamin bezeichnet. Der Einfachheit halber werden jedoch nachfolgend die Namen der Acylate von trivialen Namen abgeleitet, wobei die Lage einzelner Acylreste jeweils auf den Aminostickstoff N bzw. die als 0, 0' und 0" bezeichneten Sauerstoffatome der Hydroxylgruppen in Stellungen 6, 17, bzw. 28 bezogen wird.

Zu den markantesten Eigenschaften von Desferrioxamin B und seinen Additionssalzen, die mit einem Aequivalent Säure gebildet werden, gehört die Fähigkeit, sich zu stabilen Chelat-artigen Metallkomplexen, insbesondere mit trivalenten Metallionen, wie Chrom(III)-, Aluminium- und in erster Linie Eisen(III)-Ionen, zu verbinden. Dies verleiht Desferrioxamin B die wertvolle pharmakologische Wirksamkeit, die Ablagerung eisenhaltiger Pigmente im Gewebe zu verhindern und bei bestehenden Eisenablagerungen im Organismus eine Ausscheidung des Eisens zu bewirken, z.B. bei Hämochromatose, Hämosiderose, Lebercirrhose und Vergiftungen mit Verbindungen des dreiwertigen Eisens. Die breite therapeutische Anwendung von Desferrioxamin B und seinen Salzen (z.B. insbesondere vom Methansulfonat) erstreckt sich deshalb allgemein auf Krankheiten und krankhafte Zustände des mensschliechen Körpers (sowie des Körpers anderer Warmblüter), welcher mit übermässiger Belastung des Organismus mit Eisen(III)-Ionen ($Fe^{+++}$-Ionen) einhergehen, wie Thalassämie major, Sichelzell-Anämie, sideroachrestische Anämie, aplastische Anämie und weitere anämische Formen, in welchen Hämosiderose (d.h. eine lokale oder allgemeine Erhöhung der Eisenvorräte in sonst unbeschädigten Körpergeweben) eine Rolle spielt. Zu diesem Typus gehören auch krankhafte Zustände, die sich in Patienten nach mehrmaligen Bluttransfusionen oder mehrfach wiederholter Dialyse-Behandlung bei fehlender oder geschädigter Nierenfunktion entwickeln. Dank den komplexbildenden Eigenschaften wies Desferrioxamin B eine bedeutende Wirksamkeit bei Erkrankungen durch Eisen(III)-abhängige Mikrooganismen und Parasiten auf, wie insbesondere bei Malaria, die nicht nur in der Humanmedizin, sondern auch in der Tiermedizin von grosser Bedeutung ist. Aus die Komplex-Bildung mit anderen dreiwertigen Metallen kann zu deren Ausscheidung aus dem Organismus genützt werden, z.B. zur Entfernung von Aluminium bei der Dialyse-Encephalopathie und Osteomalcia, sowie bei der Alzheimer Krankheit.

Als ein schwerwiegender Nachteil erweist sich jedoch die Tatsache, dass Desferrioxamin und seine Salze bei oraler Gabe nur eine geringe, unzureichende Wirksamkeit aufweisen und bei allen oben genannten Anwendungsmöglichkeiten eine parenterale Verabreichungsform benötigen. So ist z.B. als eine besonders wirksame Methode empfohlen, die Wirksubstanz mittels einer langsamen (8- bis 12stündigen) subkuntanen Infusion zu verabreichen, was aber entweder eine Hospitalisierung des Patienten oder, bei ambulanter Behandlung, die Anwendung einer tragbaren mechanischen Vorrichtung, wie einer durch elektrischen Antrieb betätigten Infusionsspritze, bedingt. Abgesehen von ihrer Umständlichkeit sind solche Lösungen mit hohen Behandlungskosten behaften, was ihre Anwendung stark einschränk. Insbesondere wird eine umfassende Behandlung der Thalassämie in den Ländern des Mittelmeerraums, des Mittleren Ostens, Indiens und Südostasiens, der Malaria weltweit und der Sichelzell-Anämie in den afrikanischen Ländern verunmöglicht. Diese weit verbreiteten Krankheiten stellen weiterhin ein schwerwiegendes Problem für das Gesundheitswesen in diesen Ländern dar und machen die Suche nach einer einfacheren und billigeren Therapie, vorzugsweise mittels eines oral wirksamen Präparats, zur vordringlichen Aufgabe auf diesem Gebiet.

Auf Grund theoretischer Vorstellungen ist anzunehmen, dass zur Chelatisierung von Metallionen und somit zur therapeutisch anwendbaren Metallkomplex-Bildung die freien Amino- und Hydroxylgruppen des Desferrioxamins B den wesentlichen strukturellen Beitrag leisten. Wenn man sie aber durch Acylierung blockiert und somit ihrer Teilnahme an Komplexbildung praktisch entzieht, ist es zu erwarten, dass solche N- und/oder 0,0',0"-Acylate und andere Verbindung mit blockierten Amino- und/oder Hydroxylgruppen, wenn überhaupt, dann nur in einem sehr geringen Masse komplexbildende Eigenschaften und demzufolge auch die wesentliche Voraussetzung für die therapeutische Anwendung besitzen können.

Im Gegensatz zu diesen Ueberlegungen wurde nun gefunden, dass in denselben Indikationen, in welchen bisher Desferrioxamin B, z.B. in Form des eingeführten Handelspräparats Desferal®, nur als parenterale Darreichungsform wirksam war, die oben charakterisierte neuartige Klasse von N,N'-disubstituierten Harnstoffen der Formel I, worin $A^1$, $A^2$ und $A^3$ von Wasserstoff verschieden sind, analoge Wirkungen bei oraler Verabreichung aufweist.

Diejenigen Verbindungen der Formel I, worin $A^1$, $A^2$ und $A^3$ Wasserstoff bedeuten, sind in erster Linie parenteral wirksam. Sie sind aber Zwischenprodukte zur Herstellung derjenigen Verbindungen der Formel I, worin $A^1$, $A^2$ und $A^3$ von Wasserstoff verschieden sind. Die letztgenannten Verbindungen bewirken überraschenderweise gegenüber vorbekannten Vergleichsverbindungen bei oraler Verabreichung an Warmblüter einschliesslich des Menschen in einer Dosierung zwischen etwa 4 und 40 µmol/kg eine signifikant erhöhte Ausscheidung von Metallen, wie insbesondere Eisen. Die tägliche Dosis bei Verabreichung an einen Warmblüter von etwa 70 kg Körpergewicht beträgt etwa 0,5 g bis etwa 5 g, z.B. 2 g Wirkstoff.

Gegenstand der vorliegenden Erfindung sind in erster Linie die eingangs durch Formel I definierten N'-(Alkoxycarbonylalkyl)-harnstoff abgeleitet von Desferrioxamin B.

In den erfindungsgemässen Verbindung der Formel I können sich die Symbole $A^1$, $A^2$ und $A^3$ voneinander auch innerhalb ein und derselben Kategorie unterscheiden: So kann jedes dieser Symbole einen anderen Acylrest Ac und/oder einen anderen Carbamoylrest (II) bedeuten. Vorzugsweise haben aber alle 3 Symbole jeweils dieselbe Bedeutung und stellen insbesondere einen Acylrest Ac oder einen Carbamoylrest der Formel (II) dar, welcher vorzugsweise dieselbe Bedeutung wie B hat.

Der N-substituierte Carbamoylrest (II) ist wie folgt näher charakterisiert:

$C_2$-$C_4$-Alkyl $R^1_a$ ist vorzugsweise linear, wie insbesondere Methyl und Ethyl. $C_2$-$C_4$-Alkylen $R^1_a$ ist in erster Linie Allyl.

$C_1$-$C_7$-Alkylen Alk ist vorzugsweise $C_1$-$C_4$-Alkylen und kann beliebig verzweigt sein, wobei seine 2 freien Valenzen von zwei verschiedenen C-Atomen oder ein und demselben C-Atom ausgehen können; es kann auch einen der eingangs genannten Substituenten an einem beliebigen C-Atom tragen. Bevorzugt sind lineare Alkylenreste, die die freien Valenzen an beiden endständigen C-Atomen haben, wie Tri- bis Heptamethylen und insbesondere Ethylen. Sie können auch, vorzugsweise an ihren endständigen C-Atomen, einen Substituierten tragen, wie insbesondere Carbamoyl oder $C_1$-$C_4$-Alkoxycarbonyl (vorallem Methoxy- und Ethoxycarbonyl) oder eine primäre Aminogruppe; die zwei erstgenannten Substituenten knüpfen sich vorzugsweise an das N-terminale Ende des Alkylenrestes (d.h. an jenes, das mit der benachbarten Aminogruppe verbunden ist) an, der letztgenannte befindet sich vorzugsweise am C-terminalen Ende, d.h. an demienigen, das mit der nachfolgenden Carbonylgruppe verbunden ist. Bevorzugt sind auch lineare oder höchstens einmal verzweigte Alkylenreste, deren beide freie Valenzen von demselben C-Atom, und zwar vorzugsweise einem endständigen, ausgehen, d.h. 1,1-Alkylidenreste, wie in erster Linie Methylen, aber auch Ethyliden, 1,1-Propyliden usw. Diese können auch einen der eingangs genannten Substituenten tragen, vorzugsweise am endständigen C-Atom, wie z.B. eine freie Aminogruppe (insbesondere im 4-Amino-1,1-butyliden oder 5-Amino-1,1-pentyliden), Carbamoyl oder $C_1$-$C_4$-Alkoxycarbonyl, wie einen der obengenannten $C_1$-$C_4$-Alkoxycarbonylreste [insbesondere im 2-Carbamoyl-1,1-ethyliden, 2-(Methoxy- oder Ethoxy)-carbonyl-1,1-ethyliden oder entsprechenden 3-substituierten 1,1-Propyliden-Resten], ferner auch eine Hydroxyl- oder $C_1$-$C_4$-Alkanoyloxy (insbesondere Acetoxy)-Gruppe, welche sich vorzugsweise in 2-Stellung befindet (insbesondere im 2-Hydroxy-1,1-ethyliden und 2-Hydroxy-1,1-propyliden und entsprechenden acylierten, vor allem acetylierten Resten). Die

cyclischen Substituenten befinden sich vorzugsweise am Methylen oder auch in 2-Stellung des Ethylidenrestes.

Ein besonders bevorzugter Alkylenrest ist ein solcher, der zusammen mit der benachbarten Amino- und Carbonylgruppe durch die Teilformel -NH-Alk-CO- bzw. durch das Symbol -AAA- bezeichnet wird und welcher einerseits der allgemeinen Definition des Rests Alk, andererseits der Struktur gewisser in der Natur geläufiger α-Aminosäuren (in Form ihrer individuellen optischen Isomeren oder deren Gemische, insbesondere des razemischen Gemisches) entspricht. Der allgemeinen Konvention nach ist eine solche "geläufige α-Aminosäure" ("common α-amino acid") eine der 20 Aminosäuren, welche in der Natur regelmässig als elementare Bausteine von Peptiden und Proteinen auftreten; sie werden üblicherweise im Einklang mit der internationalen Konvention durch eine Dreibuchstaben-Kurzform bezeichnet. Von dieser Gruppe sind im vorliegenden Fall infolge der Begrenzung durch die allgemeinen Definition des Restes Alk z.B. die folgenden Säuren aus der erfindungsgemässen Bedeutung des Symbols AAA ausgenommen: Arginin (Arg), Cystein (Cys), Histidin (His) und Methionin (Met). Der obengenannte Rest -NH-Alk-CO- kann somit z.B. der bivalente Rest einer der folgenden Aminosäuren sein: Glycin (Gly), Alanin (Ala), Valin (Val), Leucin (Leu), Isoleucin (Ile), Phenylalanin (Phe), Serin (Ser), Threonin (Thr), Tryptophan (Trp), Tyrosin (Tyr), Asparagin (Asn), Glutamin (Gln) und Lysin (Lys). Entsprechende besonders bevorzugte Reste der Teilformel II sind demnach durch die Teilformel -CO-AAA-O-$R^1{}_a$ (IIA) dargestellt, worin $R^1{}_a$ die obengenannten allgemeinen und bevorzugten Bedeutungen hat und -AAA- einen durch die obige allgemeine Definition von Alk begrenzten Rest bestimmter geläufiger α-Aminosäuren in Form eines individuellen optischen Isomeren oder eines Gemisches davon bedeutet. Als optisch individuelle Form ist das "natürlich" Isomere der L-Reihe bevorzugt, als Isomerengemische sind solche bevorzugt, in welchen beide Antipoden in gleicher Menge vertreten sind, d.h. Razemate.

In erster Linie steht -AAA- für den Glycinrest (-Gly-) und der ganze Rest der Formel II ist als -CO-Gly-O-$R^1{}_a$ (IIB) hervorzuheben, in welchen $R^1{}_a$ vor allem Methyl oder Ethyl bedeutet.

Bevorzugterweise steht Alk für Ethylen, d.h. -AAA- ist der bivalente Rest von β-Alanin.

Der Acrylrest Ac leitet sich von Hydrocarbylcarbonsäuren oder Monoestern der Kohlensäure ab und entspricht der Formel Z-C(=O)-, worin Z entweder für Wasserstoff steht (und somit den Formylrest bildet) oder Hydrocarbyl R° darstellt (und somit den Rest einer gegebenenfalls substituierten acyclischen, carbocyclischen, carbocyclisch-acyclischen, heterocyclischen oder heterocyclisch-acyclischen Monocarbonsäure bildet) oder Diniederalkylamino bedeutet (und somit für den Acylrest von N-Diniederalkyl-carbaminsäure steht) oder aber Hydrocarbyloxy R°-O- ist (und den Acylrest einer einfach veresterten Kohlensäure darstellt).

Der Hydrocarbylrest (Kohlenwasserstoffrest) R° ist ein acyclischer (aliphatischer), carbocyclischer oder carbocyclisch-acyclischer Kohlenwasserstoffrest, der insgesamt vorzugsweise höchstens 40, insbesondere höchstens 20 und hauptsächlich höchstens 9, Kohlenstoffatome hat und gesättigt oder ungesättigt, unsubstituiert oder substituiert sein kann. Er kann auch anstelle von einem, zwei oder mehreren Kohlenstoffatomen gleiche oder verschiedene Heteroatome, wie insbesondere Sauerstoff, Schwefel und Stickstoff, im acyclischen und/oder cyclischen Teil enthalten; im letzteren Fall wird er als ein heterocyclischer Rest (Heterocyclylrest) oder ein heterocyclisch-acyclischer Rest bezeichnet.

Ungesättigte Reste sind solche, die einer oder mehrere Mehrfachbindungen (Doppel- und/oder Dreifachbindungen) enthalten. Cyclische Reste, worin mindestens ein 6gliedriger carbocyclischer oder ein 5- bis 8gliedriger heterocyclischer Ring die maximale Anzahl nichtkumulierter Doppelbindungen enthält, werden als aromatisch bezeichnet. Carbocyclische Reste, worin mindestens ein Ring als ein 6gliedriger aromatischer Ring (d.h. Benzolring) vorliegt, werden als Arylreste bezeichnet.

Wenn nicht anders angegeben, enthalten in der vorliegenden Offenbarung mit "nieder" bezeichnete organische Reste höchstens 7, vorzugsweise höchstens 4 Kohlenstoffatome.

Ein acyclischer Kohlenwasserstoffrest ist insbesondere ein Alkyl-, Alkenyl-, Alkadienyl- oder Alkynylrest, welcher verzweigt oder vorzugsweise linear ist.

Ein carbocyclischer Kohlenwasserstoffrest ist insbesondere ein mono-, bi- oder polycyclischer Cycloalkyl-, Cycloalkenyl- oder Cycloalkadienylrest, oder ein entsprechender, aromatische Ringe enthaltender Arylrest, vorzugsweise ein solcher mit höchstens 12 Ringkohlenstoffatomen und 5- bis 7, von allem 6gliedrigen Ringen. Carbocyclisch-acyclische Reste sind solche, in welchen ein acyclischer Reste, insbesondere einer mit höchstens 7, vorzugsweise höchstens 4 Kohlenstoffatomen, wie vor allem Methyl, Ethyl und Vinyl, einen oder mehrere carbocyclische, gegebenenfalls aromatische Reste der obigen Definition trägt.

Ein Arylrest ist in erster Linie ein Phenylrest, ferner ein Naphthylrest, wie 1- oder 2-Naphthyl, ein Biphenylylrest, wie insbesondere 4-Biphenylyl, weiter auch ein Anthryl-, Fluorenyl- oder Azulenylrest, sowie ihre Analogen mit einem oder mehreren gesättigten Ringen. Bevorzugte Aryl-niederalkyl- und niederalkenyl-Reste sind z.B. Phenylniederalkyl oder Phenyl-niederalkenyl mit endständigem Phenylrest, wie z.B. Benzyl, Phenethyl, bzw. Styryl und Cinnamyl, ferner auch o-, m-und p-Tolyl.

Heterocyclische Reste, einschliesslich heterocyclisch-acyclische Reste, sind insbesondere monocyclische, aber auch bi- oder polycyclische, aza-, thia-, oxa-, thiaza-, oxaza-, diaza-. triaza- oder tetraza-Reste aromatischen Charakters, sowie entsprechende partiell oder in erster Linie ganz gesättigte heterocyclische Reste dieser Art, wobei solche Reste gegebenenfalls, z.B. wie die obgenannten carbocyclischen oder Arylreste, weitere acyclische, carbocyclische oder heterocyclische Reste tragen können und/oder durch funktionelle Gruppen mono-, di- oder polysubstituiert sein können. Der acyclische Teil in heterocyclisch-acyclischen Resten hat z.B. die für die entsprechenden carbocyclisch-acyclischen Reste gegebene Bedeutung. Sofern sich ein Heterocyclyl als ein direkter Substituent R° im Symbol Z am Sauerstoff befindet, so muss seine freie Valenz von einem seiner C-Atome ausgehen.

Wie bereits erwähnt wurde, kann ein Hydrocarbyl (einschliesslich eines Heterocyclyls R° durch einen, zwei oder mehrere gleichartige oder verschiedenartige Substituenten (funktionelle Gruppen) substituiert sein; die folgenden Substituenten kommen insbesondere in Betracht: freie, veretherte und veresterte Hydroxylgruppen; Mercapt- sowie Niederalkylthio- und gegebenenfalls substituierte Phenylthiogruppen; Halogenatome, wie Chlor und Fluor, aber auch Brom und Iod; Oxogruppen, welche in der Form von Fomyl- (d.h. Aldehydo-) und Keto-gruppen, auch als entsprechende Acetale bzw. Ketale vorliegen; Azido- und Nitrogruppen; primäre, sekundäure und vorzugsweise tertiäre Aminogruppen, durch konventionelle Schutzgruppen geschützte primäre oder sekundäre Aminogruppen, Acylaminogruppen und Diacylaminogruppen, sowie gegebenenfalls funktionell abgewandelte Sulfogruppen, wie Sulfamoyl- oder in Salzform vorliegende Sulfogruppen. Alle diese funktionellen Gruppen dürfen sich nicht am C-Atom befinden, von dem die freie Valenz zum Sauerstoff ausgeht, vorzugsweise sind sie von dieser freien Valenz (und somit vom Heteroatom) durch 2 oder auch mehrere C-Atome getrannt. Der Hydrocarbylrest kann auch freie und funktionell abgewandelte Carboxylgruppen, wie in Salzform vorliegende oder veresterte Carboxylgruppen, gegebenenfalls einen oder zwei Kohlenwasserstoffreste tragende Carbamoyl-, Ureidocarbonyl- oder Guanidinocarbonylgruppen, und Cyangruppen tragen.

Eine als Substituent im Hydrocarbyl vorliegende veretherte Hydroxylgruppe ist z.B. eine Niederalkoxygruppe, wie die Methoxy-, Ethoxy- oder tert-Butoxygruppe, welche auch, durch Halogenatome, insbesondere in 2-Stellung, oder durch Niederalkoxyreste, insbesondere in 2-Stellung, wie im 2-Methoxyethoxyrest, substituiert sein kann. Eine besonders bevorzugte Ausgestaltung der veretherten Hydroxylgruppen liegt in Oxaalkylresten vor, in welchen ein, vorzugsweise lineares, Alkyl anstelle mehrerer C-Atome Stauerstoffatome enthält, die vorzugsweise durch mehrere (vor allem 2) C-Atome voneinander getrennt sind, so dass sie eine, gegebenenfalls mehrfach sich wiederholende Gruppe $(-O-CH_2-CH_2-)_n$, worin n = 1 bis 14, oder auch noch wesentlich mehr, bilden.

Eine als Substituent im Hydrocarbyl vorliegende veresterte Hydroxylgruppe trägt einen Acylrest Ac° mit höchstens 12 C-Atomen, welcher auch innerhalb dieser Gesamtzahl der C-Atome analog wie der Rest Ac substituiert sein kann, oder ist durch eine im Hydrocarbyl auch anwesende Carboxylgruppe lactonisiert.

Eine als Substituiert im Hydrocarby vorliegende veresterte Carboxylgruppe ist eine solche, in welcher das Wasserstoffatom durch einen der oben charakterisierten Kohlenwasserstoffreste, vorzugsweise einen Niederalkyl- oder Phenylniederalkylrest, ersetzt ist; als Beispiel einer veresterten Carboxylgruppe sind insbesondere die Methoxy-, Ethoxy-, tert-Butoxy- und Benzyloxycarbonylgruppe, sowie auch eine lactonisierte Carboxylgruppe zu nennen.

Eine bevorzugte Aminogruppe ist eine solche der Formel

$$R^1-\overset{|}{N}-R^2 ,$$

worin $R^1$ und $R^2$ unabhängig je Wasserstoff, unsubstituiertes acyclisches $C_1$-$C_7$-Hydrocarbyl (wie insbesondere $C_1$-$C_4$-Alkyl oder $C_2$-$C_4$-Alkenyl) oder monocyclisches, gegebenenfalls durch $C_1$-$C_4$-Alkyl, $C_1$-$C_4$-Alkoxy, Halogen und/oder Nitro substituiertes Aryl, Aralkyl oder Aralkenyl mit höchstens 10 C-Atomen darstellen, wobei die kohlenstoffhaltigen Reste durch eine Kohlenstoff-Kohlenstoff-Bindung oder ein Sauerstoff-, Schwefel- oder gegebenenfalls durch Hydrocarbyl substituiertes Stickstoffatom untereinander gebunden sein können. In einem solchen Fall bilden sie zusammen mit dem Stickstoffatom der Aminogruppe einen stickstoffhaltigen heterocyclischen Ring.

Ein bevorzugter Hydrocarbylrest $R^0$ im Acylrest $R^0$ -C(=O)- ist z.B. $C_1$-$C_{19}$-Alkyl oder $C_2$-$C_{19}$-Alkenyl, insbesondere ein solcher, der bei mehr als 5 C-Atomen eine lineare Kette aufweist und welcher die folgenden Substituenten tragen kann: eine Carboxylgruppe, die gegebenenfalls auch in Salzform oder als eine Cyanogruppe, eine Carbamoylgruppe oder ein $C_1$-$C_4$-Alkylester ($C_1$-$C_4$-Alkoxycarbonylgruppe) vorliegen kann und welche sich vorzugsweise in ω-Stellung befindet, eine Aminogruppe der oben definierten Formel

$$R^1-\overset{|}{N}-R^2 ,$$

oder ein oder mehrere Halogenatome, insbesondere Fluor oder Chlor, die sich vorzugsweise in der Nachbarschaft der Carbonylgruppe befinden. Ein anderes bevorzugtes Acyl dieser Art ist ein bicyclisches oder insbesondere monocyclisches Aroyl, vor allem Benzoyl, welches auch einen oder mehrere der folgenden Substituenten tragen kann: Halogenatome, insbesondere Chlor oder Fluor, Nitrogruppen, $C_1$-$C_4$-Alkylreste, insbesondere Methyl, Hydroxylgruppen und veretherte Hydroxylgruppen, insbeson-

dere $C_1$-$C_4$-Alkoxy, wie Methoxy, Phenoxy und Methylendioxy, sowie Carboxylgruppen, welche auch in der Salzform oder als eine Cyanogruppe oder ein $C_1$-$C_4$-Alkylester ($C_1$-$C_4$-Alkoxycarbonyl) vorliegen können. Vorzugsweise tragen die Aroylreste nicht mehr als 2, vor allem nur einen solchen Substituenten. Bevorzugt sind auch analoge Heteroaroylreste, insbesondere solche, die sich von Pyridin, Furan, Thiophen und Imidazol, und von ihren Analogen mit kondensiertem Benzoring (wie Chinolin, Isochinolin, Benzofuran und Benzimidazol) ableiten und gegebenenfalls auch, wie oben angegeben substituiert sind. Bevorzugt Acylreste dieser Art leite sich auch vom Benzyl und Styryl ab (d.h. Phenacetyl und Cinnamoyl), sie können auch in der oben angegebenen Weise substituiert sein.

Carbonsäure, die dem besonders bevorzugten Acylrest der Formel R°-C(=0)- zugrundeliegen, sind beispielsweise die folgenden: aliphatische Monocarbonsäuren mit höchstens 20 Kohlenstoffatomen, wie Niederalkancarbonsäuren, z.B. Propion-, Butter-, Isobutter-, Valerian-, Isovalerian, Capron-, Trimethylessig-, Oenanth- und Diethylessigsäure und vor allem Essigsäure, sowie Laurin-, Myristin-, Palmeitin- und Stearinsäure, sowie Oelsäure, Elaidinsäure, Linolsäure und Linolensäure, aber auch entsprechende halogenierte Niederalkancarbonsäure, wie die Trifluoressig-, Chloressigsäure, Bromessig- oder α-Bromisovaleriansäure, carbocyclische oder carbocyclisch-acyclische Monocarbonsäuren, z.B. die Cyclopropan-, Cyclopentan- und Cyclohexan-carbonsäure bzw. die Cyclopentan oder Cyclohexan-essigsäure oder -propionsäure; aromatische carbocyclische Carbonsäuren, z.B. Benzoesäure, die einfach oder mehrfach, wie oben angegeben, substituiert sein kann; Aryl- oder Aryloxyniederalkancarbonsäuren und deren in der Kette ungesättigte Analoga, z.B. gegebenenfalls, wie oben für die Benzoesäure angegeben, substituierte Phenylessig- bzw. Phenoxyessigsäuren, Phenylpropionsäuren und Zimtsäuren; und heterocyclische Säuren, z.B. Furan-2-carbonsäure, 5-tert-Butylfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2- oder -3-carbonsäure; ferner auch entsprechende α-Aminosäuren, insbesondere die der Ntur vorkommenden α-Aminosäuren der L-Reihe, z.B. Glycin, Phenylglycin, Prolin, Leucin, Valin, Tyrosin, Histidin und Asparagin, vorzugsweise in einer N-geschützten Form, d.h. in' einer solchen, in welcher die Aminogruppe durch eine konventionelle, z.B. eine der obengenannten, Aminoschutzgruppe substituiert ist; weiter auch Dicarbonsäure, wie Oxalsäure, Malonsäure, Mono- oder Di-niederalkylmalonsäuren, Bernsteinsäure, Glutarsäure, Adipinsäure, Erucasäure, Maleinsäure, eine durch Halogen, wie Fluor, Chlor oder Brom, und/oder Niederalkyl, Hydroxy, Niederalkoxy und Nitro gegebenenfalls substituierte Phthal-, Chinolin-, Isochinolin- oder Phenylbernsteinsäure, sowie auch Glutaminsäure und Asparaginsäure, wobei die zwei letztgenannten Säuren vorzugsweise mit geschützten Aminogruppen vorliegen. Wie bereits gesagt wurde, kann die zweite Carboxylgruppe nicht nur frei, sondern auch funktionell abgewandelt, z.B. als ein $C_1$-$C_4$-Alkylester, ein Amid oder ein Salz, vorzugsweise als ein physiologisch verträgliches Salz, mit einer salzbildenden basischen Komponente vorhanden sein. In Betracht kommen in erster Linie Metall- oder Ammoniumsalze, wie Alkalimetall- und Erdalkalimetall-, z.B. Natrium-, Kalium-, Magnesium- oder Calciumsalze, bzw. Ammoniumsalze mit Ammoniak oder geeigneten organischen Aminen.

Ein von Monoestern der Kohlensäure abgeleiteter Acylrest Ac ist durch die Teilformel R°-O-CO- charakterisiert. Als Beispiel solcher Acylreste sind diejenigen zu nennen, worin R° die folgenden bevorzugten Bedeutungen eines acyclischen Hydrocarbylrestes darstellt: $C_1$-$C_{20}$-Alkyl, $C_2$-$C_{20}$-Hydroxyalkyl, dessen Hydroxylgruppe sich in beliebiger Stellung ausser 1-Stellung, vorzugsweise in 2-Stellung, befindet, Cyano-[$C_1$-$C_{20}$]-alkyl, dessen Cyanogruppe sich vorzugsweise in 1-oder ω-Stellung befindet, oder Carboxy-[$C_1$-$C_{20}$]-alkyl, dessen Carboxylgruppe sich vorzugsweise in 1- oder ω-Stellung befindet und gegebenenfalls auch in Salzform oder als Carbamoyl bzw. $C_1$-$C_4$-Alkylester ($C_1$-$C_4$-Alkoxycarbonyl) oder Benzylester (Benzyloxycarbonyl) vorliegen kann, sowie auch ein lineares (Mono-, Di- bis Hexa)-oxaalkyl mit 4-20 Kettengliedern, worin eines oder mehrere der C-Atome, von C-3 an, enes linearen $C_4$-$C_{20}$-Alkyls durch Sauerstoffatome, welcher voneinander durch mindestens 2 C-Atome getrennt sind und vorzugsweise sich in Stellung 3-, 6-, 9-, 12-, 15- und 18- befinden, ersetzt sind.

Salze von Verbindungen der obigen Formel I mit salzbildenen Eigenschaften leiten sich in erster Linie von denjenigen ab, worin sich in einem Acylrest Ac und/oder im Rest der Formel II eine freie Aminogruppe als Substituent befindet, und sind Säureadditionssalze, insbesondere pharmazeutisch verwendbare, nicht-toxische Säureadditionssalze mit anorganischen Säuren, z.B. Chlorwasserstoffsäure, Bromwasserstoffsäure, Schwefelsäureoder Phosphorsäure, oder mit organischen Säuren wie Sulfonsäure, wie aromatischen Sulfonsäuren, z.B. Benzolsulfonsäure, p-Toluolsulfonsäure oder Naphthalin-2-sulfonsäure, oder insbesondere aliphatischen Sulfonsäuren, z.B. Methansulfonsäure, Ethansulfonsäure, 2-Hydroxyethansulfonsäure und Ethan-1,2-disulfonsäure, sowie auch Carbonsäuren, z.B. Essigsäure, Propionsäure, Glykolsäure, Bernsteinsäure, Maleinsäure, Hydroxymaleinsäure, Benzoesäure, Zimtsäure, Mandelsäure, Salicylsäure, 4-Aminosalicylsäure, 2-Phenoxybenzoesäure, 2-Acetoxy-benzoesäure, Embonsäure, Nicotinsäure oder Isonicotinsäure.

Infolge der engen Beziehung zwischen den neuen Verbindungen in freier Form und in Form ihrer Salze, inkl. auch solcher Säureadditionssalze, die als Zwischenprodukte, z.B. bei der Reinigung der neuen Verbindungen oder zu ihrer Identifikation verwendet werden können, sind vorausgehend und nachfolgend unter den freien Verbindungen sinn- und zweckgemäss gegebenenfalls auch die entsprechenden Salze zu verstehen.

Die Verbindungen der vorliegenden Erfindung weisen wertvolle Eigenschaften, in erster Linie pharmakologische Wirkungen auf, indem sie eine physiologische Wirkung zeigen, die im Grundcharakter der Wirkung des Desferrioxamins B ähnlich ist. Sie können deshalb in ähnlichen therapeutischen Indikationen wie dieses, jedoch mit dem wesentlichen Vorteil der oralen oder rektalen Verabreichung, angewendet werden, z.B. insbesondere zur Behandlung von Funktionsstörungen, in denen die Konzentration des dreiwertigen Eisens ($Fe^{3+}$-Ions) in Körperzellen übernormal hoch ist, wie bei Hämochromatose und Hämosiderose. Indem sie überdies noch in ähnlicher Weise auch Aluminium-Ionen, wie z.B. bei Dialyse-Encephalopathie, Osteomalacia und der Alzheimer-Krankheit, binden, können sie auch in diesen Indikationsbereichen mit Erfolg eingesetzt werden.

Bevorzugt sind Verbindungen der Formel I, in welcher B für einen Carbamoylrest der Teilformel II steht, worin $R^1_a$ $C_1$-$C_4$-Alkyl und Alk $C_1$-$C_4$-Alkylen bedeuten, und $A^1$, $A^2$ und $A^3$ für Wasserstoff, einen Carbamoylrest der Teilformel II, worin $R^1_a$ $C_1$-$C_4$-Alkyl und Alk $C_1$-$C_4$-Alkylen bedeuten, für Alkanoyl mit bis zu 10 C-Atomen, Niederalkoxycarbonyl, 2-(2-Methoxy-ethoxy)-ethoxycarbonyl oder Diniederalkylamino-carbonyl stehen, wobei $A^1$, $A^2$ und $A^3$ jeweils die gleiche Bedeutung haben. Von diesen Verbindungen seien besonders diejenigen Verbindungen der Formel I hervorgehoben, in welcher B Ethoxycarbonylmethylaminocarbonyl oder 2-Ethoxycarbonyl-ethylaminocarbonyl und $A^1$, $A^2$ und $A^3$ Ethoxycarbonylmethylaminocarbonyl, 2-Ethoxycarbonyl-ethylamino-carbonyl, Wasserstoff, n-Octanoyl, Ethoxycarbonyl, 2-(2-Methoxy-ethoxy)-ethoxycarbonyl oder Diethylaminocarbonyl bedeuten, wobei $A^1$, $A^2$ und $A^3$ jeweils die gleiche Bedeutung haben, insbesondere diejenigen, worin $A^1$, $A^2$ und $A^3$ Ethoxycarbonylmethylaminocarbonyl, 2-Ethoxycarbonyl-ethylaminocarbonyl, n-Octanoyl, Ethanoxycarbonyl oder 2-(2-Methoxy-ethoxy)-ethoxycarbonyl bedeuten, und diejenigen, worin $A^1$, $A^2$ und $A^3$ Diniederalkylaminocarbonyl bedeuten.

Bevorzugt sind vor allem die in den Beispielen genannten Verbindungen der Formel I.

Als eine besonders bevorzugte erfindungsgemässe Verbindung ist N,O,O′,O″-Tetra-(ethoxycarbonylmethylcarbamoyl)-desferrioxamin B der Formel I, worin jedes der Symbole B, $A^1$, $A^2$ und $A^3$ den Rest der Formel -CO-Gly-O-$R^1_a$ darstellt, in welchem $R^1_a$ Ethyl ist, hervorzuheben. Bevorzugt ist auch die analoge Verbindung, worin $R^1_a$ Methyl ist. Die erstgenannte Verbindung weist bei oraler Verabreichung an Ratten eine Wirkung auf, die mit derjenigen des parenteral verabreichten Desferrioxamins B bei gleicher Dosierung vergleichbar ist. -Besonders hevorzuheben ist auch eine Verbindung der Formel I, worin B den Rest der Formel -CO-Gly-O-$R^1_a$ darstellt, in welchem $R^1_a$ Ethyl oder auch Methyl ist, und jedes der Symbole $A^1$, $A^2$ und $A^3$ Wasserstoff oder vor allem Octanoyl, Ethoxycarbonyl oder 2-(2-Methoxyethoxy)-ethoxycarbonyl darstellt.

Ganz besonders bevorzugt sind analoge Verbindungen, worin das Symbol B für 2-Ethoxycarbonyl-ethylamino-carbonyl steht, und vor allem N,O,O′O″-tetra-(2-ethoxycarbonyl-ethylamino-carbonyl)-desferrioxamin B.

Erfindungsgemäss werden Verbindungen der Formel I unter Anwendung konventioneller, z.B. aus der Peptidchemie allgemein bekannter Analogieverfahren hergestellt, indem man

a) ein Derivat von Desferrioxamin B der Formel IV,

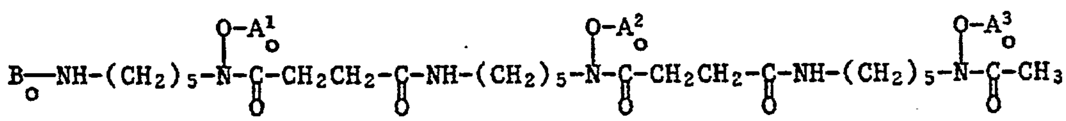

(IV)

worin $B_o$ Wasserstoff oder eine organische Silylgruppe Sil ist und jedes der Symbole $A^1_o$, $A^2_o$ und $A^3_o$ unabhängig von den anderen Wasserstoff, eine organische Silylgruppe Sil oder einen oben definierten Acylrest Ac bedeutet, mit einem Isocyanatocarbonsäureester der Formel III,

$$O{=}C{=}N\text{-}Alk\text{-}CO\text{-}O\text{-}R^1_a \qquad (III)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, oder

b) ein Derivat von Desferrioxamin B der Formel V,

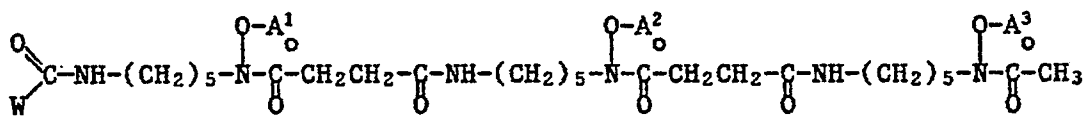

(V)

in welcher W Chlor oder 1-Imidazolyl ist, und worin jedes der Symbole $A^1_o$, $A^2_o$ und $A^3_o$ unabhängig von den anderen Wasserstoff, eine organische Silylgruppe Sil oder einen oben definierten Acylrest Ac bedeutet, mit einem Aminosäureester der Formel VI,

$$H_2N\text{-}Alk\text{-}CO\text{-}O\text{-}R^1_a \qquad (VI)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, und gegebenenfalls vorhandene N- und/oder O-Silylgruppen und/oder Aminoschutzgruppen unter Freisetzung der entsprechenden Hydroxyl- bzw. Aminogruppen abspaltet und, wenn erwünscht, eine erhaltene Verbindung der Formel I, worin mindestens eines der Symbole $A^1$, $A^2$ und $A^3$ Wasserstoff, ist zu einer Verbindung der Formel I, worin die entsprechenden Symbole für Ac oder einen Carbamoylrest der Teilformel II stehen, acyliert und/oder, wenn erwünscht, eine erhaltene freie Verbindung der Formel I mit salzbildenden Eigenschaften in ein Salz umwandelt und/oder eine Verbindung der Formel I aus einem solchen Salz freisetzt.

Die erfindungsgemässe Umsetzung des Ausgangsstoffes der Formel IV gemäss Verfahrensvariante a) mit einem Isocyanatocarbonsäureester der Formel III oder einem an der gegebenenfalls vorhandenen zusätzlichen Aminogruppe geschützten Derivat davon, wird in an sich bekannter Weise unter Anwendung konventioneller allgemeiner Methoden durchgeführt, wobei man sie durch eine geeignete Wahl der Reaktionsbedingungen so steuert, dass alle im Ausgangstoff IV vorhandenen freien Hydroxylgruppen, sowie auch die endständige freie oder silylierte Aminogruppe substituiert werden. Bei üblichen Reaktionsbedingungen wird die Silylaminogruppe durch die Carbamoylgruppe (II) acyliert, wogegen die O-Silylgruppen die O-Acylierung verhindern. Zu diesem Zweck kann man beispielsweise ein Ueberschuss am Isocyanatocarbonsäureester der Formel III unverdünnt, oder aber gelöst in einer minimalen Menge eines inerten Lösungsmittels, wie eines chlorierten Kohlenwasserstoffs (z.B. Chloroform oder Dichlormethan) einsetzen. Wenn man vom Desferrioxamin B ausgeht, verwendet man es üblicherweise in Form eines Säureadditionssalzes, insbesondere als Hydrochloride oder Methansulfonat, auch welchem die Base erst im Reaktionsgemisch mit einer aprotischen organischen Base freigesetzt wird. Die Umsetzung wird unter strengem Ausschluss von Wasser und protischer Lösungsmittel (wie insbesondere Niederalkanole) durchgeführt. Die Reaktionstemperatur liegt üblicherweise zwischen etwa 0 bis etwa 80°C, vornehmlich in der Umgebung der Raumtemperatur oder leicht oberhalb davon. Die Umsetzung erfolgt unter basischen Bedingungen und wird vornehmlich durch starke organische Basen, wie insbesondere das 1,8-Diazabicyclo[5.4.0]undec-7-en und ähnliche cyclische Basen, oder 4-Dialkylaminopyridine, z.B. 4-Dimethylamino- oder 4-Diethylaminopyridin, katalytisch beschleunigt. Zur Freisetzung des Desferrioxamins B aus der Salzform (und als zusätzliches Lösungsmittel) sind überschüssige aprotische organische Basen, wie tertiäre Amine, z.B. Triethylamin, Ethyldiisopropylamin, Tributylamin, N,N-Dimethyl- und N,N-Diethylanilin, N-Methyl- und N-Ethylpiperidin oder -morpholin und N,N'-Dimethylpiperazin, sowie stickstoffhaltige heteroaromatische Basin, wie Pyridin, Collidin und Chinnolin, geeignet.

Ueblicherweise wird die Umsetzung in Lösung oder Suspension in aprotischen inerten organischen Lösungsmitteln oder ihren zweckmässigen Gemischen, wie cyclischen Ethern (z.b. Dioxan oder Tetrahydrofuran), tertiären Amiden (z.B. N,N-Dimethylformamid, N,N-Dimethylacetamid, N-Methylpyrrolidon und Hexamethylphosphortriamid), Dimethylsulfoxid, sowie auch tertiären Aminen (z.B. den obengenannten) oder Acetonitril oder ähnlichen Niederalkylcyaniden durchgeführt. Zum besseren Vermischen von spärlich löslichen oder miteinander mischbaren Komponenten arbeitet man vorzugsweise unter intensivem Rühren.

Verbindungen der Formel I, worin B eine andere Bedeutung als $A^1$, $A^2$ und $A^3$ hat, kann man vorteilhaft herstellen, indem man von einer Verbindung der Formel IV ausgeht, worin $B_o$ sowie mindestens eines der Symbole $A^1_o$, $A^2_o$ und $A^3_o$ (vorzugsweise aber alle) für eine organische Silylgruppe (Sil) der Formel

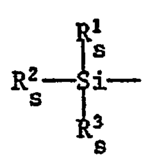 (Sil),

stehen, in welcher $R^1_s$ und $R^2_s$ unabhängig je unsubstituiertes $C_1$-$C_8$-Hydrocarbyl und $R^3_s$ unsubstituiertes $C_1$-$C_8$-Hydrocarbyl oder Chlor bedeutet.

Die in der organischen Silylgruppe Sil vorhandenen Hydrocarbylreste $R^1_s$ und $R^2_s$ sind insbesondere $C_1$-$C_8$-Alkylreste, beispielsweise Hexyl, 4-Methylpentyl, Pentyl, Ethyl und vor allem Methyl, ferner auch Aryl- und Aralkylreste, beispielsweise Phenyl oder p-Tolyl, bzw. Benzyl oder Phenethyl; vorzugsweise sind beide Reste gleich. Das Symbol $R^3{}_S$ kann für Chlor stehen oder eine der genannten Bedeutungen der Symbole $R^1{}_S$ und $R^2{}_S$ haben, wobei alle 3 Symbole vorzugsweise dieselbe Bedeutung haben; und vor allem bedeutet $R^3{}_S$ Methyl.

Eine geeignete organische Silylgruppe Sil ist beispielsweise Trimethylsilyl, Tribenzoylsilyl, Phenyl-dimethylsilyl, Benzyl-dimethylsilyl, Hexyl-dimethylsilyl, tert-Butyl-dimethylsilyl, Triethylsilyl, Diethyl-chlorsilyl und insbesondere Dimethyl-chlorsilyl und vor allem Trimelthylsilyl.

Die Umsetzung eines solchen (N- und O-)-silylierten Ausgangsstoffes mit dem Reagens der Formel III wird vorzugsweise auch unter den oben geschilderten allgemeinen Bedingungen durchgeführt, wobei nur die N-silylierte Aminogruppe durch den Rest (II) selektiv substituiert wird, wogegen die O-gebundenen Silylgruppen bestehen bleiben und in der anschliessenden Solvolyse zusammen mit der N-Silylgruppe (unter Freisetzung der Hydroxylgruppe und der Acylaminogruppe) entfernt werden. Die Solvolyse kann in konventioneller Weise mit einem protischen Reagens (einschliesslich Wasser), welches zugleich als Lösungsmittel dienen kann, und vorzugsweise unter Säurekatalyse durchgeführt werden. Vorteilhaft wird dem Reaktionsgemisch nach der Hauptreaktion ein Niederalkanol, wie Ethanol oder vor allem Metha nol, (und gegebenenfalls eine starke Säure, wie Chlorwasserstoff) zugesetzt, wodurch die abgespaltenen Silylgruppen zu leicht flüchtigen, abdestillierbaren niederaliphatischen Silylethern umgewandelt werden.

Vornehmlich kann man die oben beschriebenen silylierten Ausgangsstoffe der Formel IV direkt im Reaktionsmedium in situ bilden, indem man Desferrioxamin B oder ein Säureadditionssalz davon (bzw. ein teilweise 0-acyliertes Analoges) in Gegenwart einer aprotischen organischen Base wie einer oben genannten, vor allem Pyridin, (welche zugleich als Lösungsmittel dienen kann) mit einem Silylierungsreagens, insbesondere einem Silylhalogenid der Formel Sil-Hal, worin Sil die oben genannte Bedeutung hat und Hal für Brom oder insbesondere Chlor steht, umsetzt. Ein besonders bevorzugtes Silylierungsreagens ist z.B. ein Triniederalkylsilylchlorid, wie Trimethylsilylchlorid, oder auch ein Diniederalkyldichlorsilan, wie Dimelthyldichlorsilan. Vornehmlich wird das Silylierungsmittel in überschüssiger Menge zugesetzt; seine Gegenwart stört oder beeinträchtigt die Hauptreaktion (das Umsetzen mit einem Isocyanat der Formel III) in keiner Weise, im Gegenteil, sie entfernt jede mögliche Spur von störender Feuchtigkeit. Deshalb kann die Hauptreaktion im Anschluss an die Silylierung im gleichen Reaktionsmedium vorgenommen werde und sogar mit der nachfolgenden solvolytischen Abspaltung der Silylgruppen zusammengelegt werden, so dass alle 3 Stufen (Herstellung des Ausgangsstoffes, Behandeln mit Isocyanat und Abspalten der Silylgruppen) in ein und demselben Reaktionsmedium durchgeführt werden können. Das überschüssige Silylierungsreagens, sowie auch das überschüssige Isocyanat, werden dabei vorteilhaft unter den Bedingungen der Solvolyse zerstört und in flüchtige Produkte umgewandelt; die Silylhalogenide liefern dabei Chlorwasserstoff, der die Solvolyse vorteilhaft katalysiert.

Auch Variante b) des erfindungsgemässen Verfahrens wird in einer an sich bekannten allgemeinen Weise durchgeführt, wobei die Umsetzung des N-Chloroformyl - bzw. N-(1-Imidazolyl-carbonyl)-derivats von Desferrioxamin B mit einem Aminosäureester der Formel VI unter analogen Bedingungen und in denselben Lösungsmittel wie die Verfahrensvariante a) durchgeführt werden kann. Im Aminosäureester VI muss eine gegebenenfalls vorhandene zusätzliche Aminogruppe in geschützter Form voliegen, vorzugsweise können auch Carboxylgruppen in Esterform vorliegen. die Verbindung kann auch als ein Säureadditionssalz eingesetzt werden, in welchem Falle die reaktive freie Form der Aminogruppe erst durch die Einwirkung der als Reaktionsmedium verwendeten organischen Base, wie Pyridin, gebildet wird. - Die Ausgangsstoffe der Formel IV, worin B für Chloroformyl bzw. die 1-Imidazolylcarbonylgruppe steht und $A^1{}_o$, $A^2{}_o$ und $A^3{}_o$ unabhängig voneinander Wasserstoff, die Silylgruppe Sil oder den Acylrest Ac bedeuten, können in an sich bekannter allgemeiner Weise, z.B. durch das oben geschilderte Verfahren, hergestellt werden. So kann man beispielsweise Desferrioxamin B oder ein Säureadditionssalz davon (oder ein teilweise O-acyliertes Analoges) in Gegenwart einer aprotischen organischen Base (wie einer oben beschriebenen) mit einem Silylierungsreagens (wie einem der oben genannten) behandeln und das erhaltene (N- und O-)-silylierte Derivat mit Phosgen bzw. Bis-(1-imidazolyl)-carbonyl, vorzugsweise mit einem molaren Aequivalent davon, umsetzen. Vornehmlich führt man beide Reaktionen nacheinander im demselben Medium durch. Das erhaltene N-Chloroformyl- bzw. N-(1-Imidazolylcarbonyl)-derivat kann man entweder einer vorsichtigen solvolytischen Abspaltung der Silylgruppen unterwerfen und in individuller Form (gewünschtenfalls, nach Acylieren als ein O-Acylat) isolieren, oder aber noch in silylierter Form mit dem Aminosäureester VI umsetzen, und erst nachher die Silylgruppen entfernen. Alle Stufen erfolgen unter den oben beschriebenen allgemeinen Bedingungen, vorzugsweise in Pyridin oder einer ähnlichen heterocyclischen Base, welche sowohl als Lösungsmittel wie auch das basische Reagens dient.

Zum vorübergehenden Schutz einer gegebenenfalls vorhanden Aminogruppe während der erfindungsgemässe Umsetzung eignen sich die üblichen Aminoschutzgruppen, welche bei der Synthese der Peptidkette verwendet werden und einschliesslich entsprechender Abspaltungsmethoden in Uebersichtsreferaten und Nachschlagewerken, wie Houben-Weyl: Methoden der organischen Chemie; 4. Auflage, Band 15/I und II, E. Wünsch (Herausgeber): Synthese von Peptiden (Georg-Thieme Verlag, Stuttgart; 1974), ausführlich beschrieben sind. Vorzugsweise werden acidolytisch oder neutral abspaltbare Aminoschutzgruppen verwendet.

Unter geeigneten Aminoschutzgruppen kommt z.B. durch Methyl, Methoxy, Halogene und/oder Nitro substituiertes Trityl in Betracht, oder vorzugsweise die unsubstituierte Trityl- (Triphenylmethyl-) Gruppe, welche unter sehr milden Bedingungen, wie bereits mit ca. 50%iger Essigsäure, solvolytisch (acidolytisch) abspaltbar ist. - Auch im Ring substituierte Phenylsulfonylgruppen, vor allem die 2-Nitrophenylsulfenylgruppe o-$O_2N$-$C_6H_4$-S-, sind zu erwähnen; die letztgenannte ist z.B. durch eine säurekatalysierte Solvolyse oder Acidolyse, z.B. bereits mit Pyridinhydrochlorid, abspaltbar.

Als wichtigste Aminoschutzgruppen kommen jedoch veresterte Oxycarbonylreste der Teilformel $R_o$-O-CO- in Frage, worin $R_o$ ein neutral und/oder acidolytisch abspaltbarer Hydrocarbylrest ist.

Derartige Aminoschutzgruppen sind beispielsweise gegebenenfalls im aromatischen Ring durch Halogenatome, Nitrogruppen, Niederalkyl- oder Niederalkoxygruppen substituierte Benzyloxycarbonylgruppen, wie unsubstituiertes Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl und p-Tolyloxycarbonyl, bzw. Furfuryloxycarbonyl, sowie auch 2-(4-Biphenylyl)-2-propyloxycarbonyl und ähnliche im Schweizer Patent 509 266 beschriebene Aralkoxycarbonylreste. Diese Reste lassen sich, wie noch weitere unten näher beschrieben wird, unter neutralen Bedingungen hydrogenolytisch, oder aber vorzugsweise acidolytisch abspalten.

Ein weiterer solcher Acylrest $R_o$-O-CO- ist beispielsweise vor allem tert.-Butoxycarbonyl, oder auch ein analoger Rest, wie Isopropyloxycarbonyl, tert.-Amyloxycarbonyl (d.h. 1,1-Dimethylpropyloxycarbonyl), Diisopropylmethoxycarbonyl, Cyclopentyloxycarbonyl, Cyclo hexyloxycarbonyl, d-Isobornyloxycarbonyl und Adamantyloxycarbonyl. Diese Reste lassen sich, wie noch weiter unten näher beschrieben wird, vornehmlich unter sauren Bedingungen (acidolytisch) abspalten.

Ein noch weiterer Acylrest $R_o$-O-CO-, ist z.B. ein β-(Trihydrocarbylsilyl)-ethoxycarbonylrest, wie β-(Triniederalkylsilyl)-ethoxycarbonyl, z.B. insbesondere β-(Trimethylsilyl)-ethoxycarbonyl. Solche Reste bilden mit der zu schützenden Aminogruppe entsprechende β-Trihydrocarbylsilyl-ethoxycarbonylaminogruppen (z.B. die β-Trimethylsilylethoxycarbonylaminogruppe), welche unter den Bedingungen der sauren Hydrolyse und der Hydrogenolyse zwar beständig sind, aber unter ganz spezifischen, sehr milden Bedingungen durch Einwirkung von Fluoridionen sich Abspalten lassen.

Besonders hervorzuheben ist auch Allyloxycarbonyl, welches sich nicht nur acidolytisch, sondern besonders auch unter sehr milden neutralen Bedingungen mit Dimedon, oder durch die spezifische reduktive Einwirkung von Tributylzinnhydrid unter Katalyse mit Palladium-(O)-tetrakis-(triphenylphosphin)-Komplex abspalten lässt.

Die erfindungsgemässe nachträgliche Abspaltung der Aminoschutzgruppe erfolgt in der allgemein bekannten Weise, wobei spezifische Bedingungen für einzelne Strukturtypen in der einschlägigen Literatur (siehe z.B. Houben-Weyl, loc. cit.) bis ins Detail beschrieben sind. Die Acidolyse (einschliesslich saurer Hydrolyse) wird z.B. mit Trifluoressigsäure, Fluorwasserstoff, Bromwasserstoff und Chlorwasserstoff gegebenenfalls in Anwesenheit von Wasser, wie mit Salzsäure, und bei säureempfindlichen Schutzgruppen auch mit einer niederaliphatischen Carbonsäure, wie Ameinsensäure und/oder Essigsäure, gegebenenfalls in Anwesentheit von Wasser durchgeführt. Die neutral abspaltbaren Gruppen, insbesondere solche, die Benzylreste enthalten, werden vorzugsweise hydrogenolytisch, z.B. durch Hydrierung unter Palladium-Katalyse, entfernt. Die β-Silylethoxycarbonylgruppen werden vorzugsweise mit Fluoridionen-abgebenden Mitteln, z.B. mit Fluoriden quaternärer organischer Basen, wie Tetraethylammoniumfluorid oder Tetrabutylammoniumfluorid, in neutralen organischen Lösungsmitteln abgespalten.

Je nach Arbeitsweise erhält man solche Endstoffe mit unsubstituierter Aminogruppe in Form von Basen oder Säureadditionssalzen. Aus den Säureadditionssalzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen.

Gewünschtenfalls kann man in erhaltenen Endstoffen der Formel I, worin mindestens eines (und vorzugsweise alle) der Symbole $A^1$, $A^2$] und $A^3$ Wasserstoff bedeutet, die entsprechenden freien Hydroxylgruppen acylieren, indem man eine solche Verbindung mit einem den Acylrest Ac oder einen Carbamoylrest der Formel II einführenden Mittel behandelt.

Als einen Acylrest Ac einführende Mittel werden die üblichen, zu diesem Zweck allgemein gebräuchlichen Acylierungsmittel verwendet; insbesondere verwendet man Acylierungsmittel der Formel AcY, worin Ac die oben angegebenen allgemeinen und hervorgehobenen Bedeutungen hat, und Y eine reaktionsfähige funktionell abgewandelte Hydroxylgruppe ist oder ein zusätzliche einfache Bindung zum Rest Ac darsellt, deren anderes Ende ein Wasserstoffatom im Rest Ac ersetzt.

Als ein den Carbamoylrest der Formel II einführendes Mittel ist z.B. ein oben definierter Carbonylaminosäureester der Formel III zu nennen, mit welchem die Acylierung unter den oben angegebenen allgemeinen Bedingungen erfolgt. Ein Acylierungsmittel, das sich vom oben definierten Acylrest Ac ableitet, ist insbesondere ein solches, worin Y eine veresterte Hydroxylgruppe ist, beispielsweise eine solche, die mit einer starken anorganischen Säure, wie einer Halogenwasserstoffsäure, (z.B. Chlor-, Brom- oder Iodwasserstoffsäure), einer Pseudohalogenwasserstoffsäure, wie Azoimid oder Imidazol (unter Abspaltung des H-Atoms von 1-N-Atom), einer sauerstoffhaltigen Mineralsäure, wie Phosphorsäure und insbesondere Schwefelsäure, oder einer starken organischen, wie aliphatischen oder aromatischen Sulfonsäure, (z.B. Methan- und Ethan- bzw. Benzol-, p-Toluol-, p-Nitrobenzol- und p-Chlorbenzolsulfonsäure) verestert wird. Eine derartige veresterte Gruppe bildet dann mit dem Acylrest ein gemischtes Anhydrid. Darunter besonders hervorzuheben sind gemischte Anhybride mit Halogenwasserstoffsäuren und Pseudohalogenwasserstoffsäuren, wie Säurebromide, Säurechloride, Säureazide und 1-Imidazolyl-Derivate der Formel $R^o$-CO-Hal bzw. $R^o$-O-CO-Hal, worin Hal Brom oder Azido und vorzugsweise Chlor oder 1-Imidazolyl bedeutet und $R^o$ die oben genannten Bedeutungen hat. Als ein Reagens dieses Typs, welches insbesondere bei Herstellung von Ausgangsstoffen für die Verfahrensvariante b) von Wichtigkeit ist, sind Phosgen und sein weniger toxisches Analogon Bis-(1-imidazolyl)-carbonyl (und ähnliche Reagentien) zu erwähnen. Diese setzt man in der Regel in äquimolaren Mengen ein, so dass die zweite reaktionsfähige Gruppe Y im Produkt erhalten bleibt und nachträglich abgewandelt werden kann.

Als Beispiel für ein Acylierungsmittel AcY zur Einführung eines Diniederalkylamino-carbonylrestes, z.B. des Diethylamino-carbonylrestes, sei Diniederalkyl-carbamoylchlorid, z.B. Diethylcarbamoylchlorid, genannt.

Die reaktionsfähige veresterte Hydroxylgruppe kann aber auch entweder durch den Rest einer anderen Carbonsäure, insbesondere einer stärkeren Carbonsäure, wie der Ameisensäure, Chloressigsäure oder vornehmlich der Trifluoressigsäure, verestert werden und einem gemischten Anhydrid zugrundeliegen, oder aber durch denselben Acylrest verestert werden und ein symmetrisches Carbonsäureanhydrid der Formel $Ac^1$-O-$Ac^1$, insbesondere eines der Formeln $R^o$-CO-O-CO-$R^o$ oder $R^o$-O-CO-O-CO-O-$R^o$ bilden.

Acylierungsmittel der Formel AcY, in welchen Y eine zusätzliche Bindung zum Rest Ac darstellt, leiten sich insbesondere von Acylresten der Carbonsäuren, die am C-Atom, das mit der Carboxylgruppe benachbart ist, einen Wasserstoff tragen; sie gehören der Kategorie der Ketene der Formel $R^a_o$=C=O, worin $R^a_o$ für Hydrocarbyliden steht, d.h. für einen zweiwertigen, dem Rest $R^o$ entsprechenden Rest aliphatischen Charakters, in welchem das funktionalisierte Kohlenstoffatom durch einfache Bindungen mit benachbarten Kohlenstoff- und/oder Wasserstoffatomen verbunden ist.

Die Umsetzung mit dem Acylierungsmittel der Formel AcY erfolgt unter bekannten Verfahrensbedingungen, die in der organischen Chemie allgemein für die Acylierung von Hydroxyverbindungen gebräuchlich sind, üblicherweise bei Temperaturen zwischen dem Gefrierpunkt und dem Siedepunkt des Reaktionsgemisches, wie im Temperaturbereich von etwa -10 bis etwa +160°, insbesondere von etwa +20 bis etwa +50°, beim atmosphärischen oder erhöhten Druck, in heterogener Phase (wie Suspension) unter Rühren oder Umschütteln, oder vornehmlich in homogener flüssiger Phase, wie in einem Ueberschuss von flüssigem Reagens oder insbesondere in Anwesenheit von Lösungsmitteln, insbesondere organischen Lösungsmitteln, und gegebenenfalls in Gegenwart von säurebindenden anorganischen oder organischen Mitteln. Geeignete Lösungsmittel sind beispielsweise aprotische organische Lösungsmittel niedriger Polarität, wie halogenierte, insbesondere chlorierte, aliphatische Kohlenwasserstoffe, wie Chloroform und Dichlormethan, und insbesondere polare aprotische Lösungemittel, wie aliphatische und cyclische Ether, z.B. Diethylether, 1,2-Dimethoxyethan und Diisopropylether bzw. Dioxan und Tetrahydrofuran, niederaliphatische Ester und Amide, wie Ethylacetat bzw. Formamid, Acetamid, N,N-Dimethylacetamid und Dimethylformamid, sowie Acetonitril, Dimethylsulfoxid und Hexamethylphosphortriamid; die Lösungsmittel können auch in zweckmässigen Kombinationen, z.B. zur Erhöhung der Löslichkeit von Komponenten, eingesetzt werden.

Als säurebindende Mittel können im Prinzip beliebige basische Verbindungen zugezogen werden, wie einerseits organische stickstoffhaltige Basen, z.B. tertiäre Amine vom Typ Triethylamin, Ethyldiisopropylamin, N,N-Dimethylanilin, N-Ethylpiperidin oder N,N'-Dimethylpiperazin, oder aromatische heterocyclische Basen von Typ Pyridin, Collidin, Chinolin oder 4-Dimethylaminopyridin, andererseits basisch reagierende anorganische Verbindungen, wie Alkalimetallhydroxide, -carbonate und -hydrocarbonate, sowie Salze von Carbonsäuren, wie Natrium- oder Kaliumacetat. Schliesslich können, diese Rolle auch neutral reagierende stickstoffhaltige Verbindungen übernehman, die zugleich oft auch vorteilhafte Lösungsmittel darstellen z.B. Carbonsäureamide, insbesondere niederaliphatische Carbonsäureamide, wie die oben genannten, und cyclische Amide, wie N-Methylpyrrolidon, sowie Amidoderivate der Kohlensäure, wie Urethane und Harnstoff. Umgekehrt können die oben erwähnten Basen, insbesondere die von Typ Pyridin, als Lösungsmittel dienen.

Falls der Hydrocarbylrest $R^o$ durch funktionelle Gruppen substituiert ist, die während der Acylierung mitreagieren könnten, wie frei Carboxyl-, Hydroxyl- und insbesondere Aminogruppen, werden diese vornehmlich vorübergehend geschützt, oder vorzugsweise im angewendeten Acylierungsmittel bereits in geschützter Form vorliegen, und nach erfolgter Acylierung von diesen Schutzgruppen befreit.

So z.B. gehört zu den gewöhnlichsten Methoden zum Schutz von Carboxylgruppen die Veresterung. Die Freisetzung einer veresterten Carboxylgruppe erfolgt im allgemeinen durch konventionelle Hydrolyse, vor allem unter Einwirkung von Basen (wie vornehmlich von Alkalimetall-hydroxiden, -carbonaten der -hydrocarbonaten), oder aber, bei geeigneten Estern, wie solchen von tertiären Alkoholen (z.B. tert-Butylalkohol), durch Acidolyse, z.B. mittels Fluorwasserstoff oder Trifluoressigsäure. Ester mit Benzylalkoholen können auch durch konventionelle Hydrogenolyse abgespalten werden.

Die zum vorübergehenden Schutz von Hydroxylgruppen anzuwendenden Gruppen und Abspaltungsmethoden sind auch allgemein bekannt, z.B. aus der Synthese von Peptiden. Insbesondere schützt man Hydroxylgruppen in der Form von Estern mit Carbonsäuren, wie mit Niederalkansäuren oder mit Monoestern der Kohlensäure (z.B. Formiate oder Acetate einerseits oder tert-Butoxy- oder Benzyloxy-carbonate andererseits), oder aber in der Form von Ethernm, wie insbesondere solchen von tertiären Alkoholen (z.b. tert-Butylalkohol), oder auch in der Form von Acetalen (z.B. insbesondere als 2-Tetrahydropyranylether). Die ersteren Schutzgruppen werden üblicherweise analog wie veresterte Carboxylgruppen abgespalten; beide letzteren werden vornehmlich durch Acidolyse entfernt.

Die zum vorübergehenden Schutz von primären und sekundären Aminogruppen verwendbaren Schutzgruppe entsprechen denjenigen, die oben beim Hauptverfahren eingehend diskutiert wurden.

Die wichtigsten Ausgangsstoffe, insbesondere Desferrioxamin B und seine Säureadditionssalze einerseits, oder die N-Carbonylaminosäureester, insbesondere die von geläufigen α-Aminosäuren abgeleiteten, andererseits, sind bekannt oder naheliegend. Sofern sie unbekannt sind, wie einige zur Acylierung zu verwendenden Isocyanatoalkansäureester (Carbonylaminosäureester), können in an sich bekannter, konventioneller Weise, z.B. durch Behandeln eines Aminosäureesters in Form eines Säureadditionssalzes davon, und gegebenenfalls unter Schutz einer als Substituent vorhandenen, nicht umzusetzenden Aminogruppe mit einer mindestens äquivalenten, vorzugsweise überschüssigen Menge Phosgen, gegebenenfalls in Anwesenheit eines nicht-acylierbaren Amins (wie eines oben genannten) erhalten werden. Erhaltene Verbindungen mit geschützter Aminogruppe werden vorteilhaft als solche in der Hauptreaktion eingesetzt und die Schutzgruppe erst nachträglich abgespalten.

Beim Verfahren der vorliegenden Erfindung werden vorzugsweise solche Ausgangsstoffe verwendet, welche zu den eingangs als besonders wertvoll geschilderten Verbindungen führen.

Die Erfindung Betrifft auch diejenigen Ausführungsformen des Verfahrens, bei denen man ein Ausgangsstoff unter den Reaktionsbedingungen gebildet oder in Form eines Derivats davon, zum Beispiel eines Salzes, verwendet wird.

Die vorliegende Erfindung betrifft ebenfalls pharmazeutische Zuammensetzungen, die als Wirkstoff eine der neuen pharmakologisch wirksamen Verbindungen der Formel I, insbesondere eine der oben für diese Anwendung hervorgehobenen, enthalten. Besonders bevorzugt sind Präparate und Zusammesetzungen zur enteralen, wie insbesondere oralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab, im allgemeinen entspricht sie jedoch mengenmässig etwa derjenigen für parenteral appliziertes Desferrioxamin B oder ein Salz davon.

Die pharmazeutischen Zusammensetzungen enthalten vorzugsweise von etwa 5 % bis etwa 95 % des Wirkstoffs, wobei einzeldosierte Applikationsformen vorzugsweise von etwa 20 % bis etwa 90 % und nichteinzeldosierte Applikationsformen vorzugsweise etwa 5 % bis etwa 20 % Wirkstoff aufweisen; pharmazeutische Präparate in Dosiseinheitsform, wie Dragées, Tabletten oder Kapseln, bzw. Suppositorien enthalten von etwa 0,1 g bis etwa 3,0 g vorzugsweise von etwa 0,3 g bis etwa 1,0 g des Wirkstoffs.

Die pharmazeutischen Zusammensetzungen der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol.

Dragées-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Zusammensetzungen sind Steckkapseln aus Gelatine, sowie weiche, geschlossene Kapseln aus Gelatine und einem Weichmacher, wie Glycerine oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei

ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Die Erfindung betrifft ebenfalls ein Verfahren zur Behandlung von Krankheiten, bei denen, wie oben beschrieben worden ist, ein Ueberschuss von Eisen(III) oder Aluminium im Körper vorhanden ist, dadurch gekennzeichnet, dass man eine prophylaktisch oder therapeutisch wirksame Menge einer Verbindung der Formel I, vorzugsweise peroral, verabreicht. Dabei verwendet man in erster Linie die obengenannten pharmazeutischen Zusammensetzungen, wobei man einem Warmblüter von etwa 70 kg Gewicht eine tägliche Dosis von etwa 0,5 g bis etwa 15 g, vorzugsweise von etwa 1,5 g bis etwa 7,5 g einer Verbindung der vorliegenden Erfindung verabreicht.

Die nachfolgenden Beispiele illustrieren die vorliegende Erfindung; Temperaturen werden in Celsiusgraden angegeben.

<u>Beispiel 1:</u> N,O,O',O"-Tetra-(ethoxycarbonylmethylcarbamoyl)-desferrioxamin B

Unter striktem Ausschluss von Feuchtigkeit wird eine intensiv gerührte Suspension von 6,56 g (0.01 Mol) Desferrioxamin B - methansulfonat in 200 ml Acetonitril, 200 ml Dichlormethan und 20 ml Dimethylsulfoxid bei Raumtemperatur nacheinander mit 14 ml (0,1 Mol) Triethylamin, einer Lösung von 13 g (0,1 Mol) Isocyanatoessigsäureethylester (Ethyl-N-carbonylglycinat) in 100 ml Dichlormethan, und 50 mg 4-Dimethylaminopyridin (als Katalysator) versetzt und bei Raumtemperatur gerührt, bis alle festen Anteile in Lösung übergehen (3–4 Stunden). Das Reaktionsgemisch wird nach einer zusätzlichen Stunde im Vakuum von flüchtigen Anteilen befreit, der schwach saure (pH etwa 5) Rückstand in 300 ml Wasser aufgenommen, mit 1-N Natriumhydroxid auf pH = 7,7 gebracht und dreimal mit je 500 ml Dichlormethan extrahiert. Die vereinigten organischen Auszüge werden mehrmals mit Wasser gewaschen, über Natriumsulfat getrocknet und im Vakuum vom Lösungsmittel befreit. Der feste amorphe Rückstand stellt die rohe Titelverbindung in brauchbarer Reinheit dar. Zur weiterer Reinigung kann er an Silicagel mit Gemischen von Dichlormethan/Tetrahydrofuran/2-Propanol im Verhältnis von etwa 85:15:0 bis 80:15:5 chromatographiert werden. Das schaumartige, praktisch farblose Produkt weist bei der massenspektroskopischen Molekulargewichtsbestimmung den Wert von $MH^+$ = 1078 auf, welcher mit dem für die Formel $C_{45}H_{76}N_{10}O_{20}$ berechneten Wert 1077,153 übereinstimmt; $R_f$ = 0,24 (Dichlormethan:2-Propanol = 85:15), $R_f$ = 0,31 (Dichlormethan:Methanol = 90:10), $R_f$ = 0,59 (Dichlormethan:Methanol = 85:15),

360 MHz-$^1$H-NMR, DMSO-$d_6$, 40°C: $\delta$ = 1.19 und 1.26 und 1.38 und 1.51 (m; 30 H), 1.93 (s; 3 H), 2.30 (t; 4 H), 2.48 (t; 4 H), 3.00 (m; 6 H), 3.54 (t; 6 H), 3.74 (d; 2 H), 3.82 (d; 6 H), 4.11 (m; 8 H), 6.02 (breit; 2 H), 7.63 (breit; 3 H), 8.23 (breit; 2 H).

In analoger Weise kann auch eine äquivalente Menge (5,97 g) von Desferrioxamin B-hydrochlorid zum identischen Endprodukt umgewandelt werden.

<u>Beispiel 2:</u> N-(Ethoxycarbonylmethylcarbamoyl)-desferrioxamin B.

Eine Suspension von 26,3 g (40 mMol) Desferrioxamin B-methansulfonat in 300 ml Pyridin wird während 10 Minuten mit 50,0 ml (400 mMol) Trimethylchlorsilan (TMS) versetzt und während 3 Stunden bei Raumtemperatur gerührt. Die Reaktionslösung wird bei 22° während 10 Minuten mit 9,3 g (72 mMol) Isocyanatoessigsäure-ethylester versetzt und weitere 6 Stunden bei Raumtemperatur gerührt. Durch Zugabe von 150 ml Methanol werden überschüssige Reagentien zerstört und die Silylgruppen abgespalten, und Lösungsmittel werden durch Destillation entfernt. Der feste Rückstand wird im Hochvakuum getrocknet und zuerst aus Wasser, dann aus Methanol/Dichlormethan kristallisiert; Smp. 177-178°.

<u>Beispiel 3:</u> N-(Ethoxycarbonylmethylcarbamoyl)-O,O',O"-trioctanoyl-desferrioxamin B der Formel I, worin B = $C_2H_5O$-CO-$CH_2$-NH-CO- und $A^1$ = $A^2$ = $A^3$ = $CH_3$-$(CH_2)_6$-CO- ist.

In eine Suspension von 6,9 g (10 mMol) N-(Ethoxycarbonylmethylcarbamoyl)-desferrioxamin B (aus Beispiel 2) in 120 ml Pyridin werden unter Argon bei 23° im Laufe von 5 Min. 5,65 ml (33 mMol) Octanoylchlorid zugesetzt. Die Reaktionslösung wird 5 Stunden bei Zimmertemperatur gerührt, das überschüssige Acylierungsmittel durch Zugabe von 100 ml Methanol zerstört, und die flüchtigen Anteile durch Abdestillieren entfernt. Der Rückstand wird in 350 ml Phosphatpuffer von pH = 7,4 aufgenommen und mit Dichlormethan extrahiert. Nach Trocknen wird die Dichlormethan-Lösung zweimal über Kieselgel chromatographiert; Elution mit Gemischen von Dichlormethan-Isopropylalkohol (V/V-Verhältnis von 97 : 3 bis 94 : 6) ergibt das Produkt als farbloses Oel, welches bei Raumtemperatur kristallin erstarrt. Das Produkt ergibt eine Elementaranalyse, die der Theorie für die Titelverbindung $C_{54}H_{97}N_7O_{14}$ (1068,4) entspricht; Smp. 65°C.

Beispiel 4: N-(Ethoxycarbonylmethylcarbamoyl)-O-O'-O"-tri[2-(2-methoxyethoxy)-ethoxycarbonyl]-desferrioxamin B der Formel I, worin B = $C_2H_5O$-CO-$CH_2$-NH-CO- und $A^1$ = $A^2$ = $A^3$ = $CH_3$-O-$CH_2$-$CH_2$-O-$CH_2$-$CH_2$O-CO- ist.

In eine Suspension von 6,9 g (10 mMol) N-(Ethoxycarbonylmethylcarbamoyl)-desferrioxamin B (aus Beispiel 2) in 120 ml Pyridin wird unter Argon bei 23° im Laufe von 5 Min. eine Lösung von 7,86 g (43 mMol) von 2-(2-Methoxyethoxy)-ethyl-chlorameisensäureester in 40 ml Toluol zugetropft. Das Reaktionsgemisch wird 17 Stunden bei Zimmertemperatur gerührt, das überschüssige Acylierungsmittel durch Zugabe von 160 ml Methanol zerstört, und die flüchtigen Anteile durch Abdestillieren entfernt. Der Rückstand wird in 350 ml Phosphatpuffer von pH = 7,4 aufgenommen und mit Dichlormethan extrahiert. Nach Trocknen wird die Dichlormethan-Lösung zur Trockne abgedampft, in einer minimalen Menge Heptan-Dichlormethan (1:2) gelöst und über Kieselgel chromatographiert; Elution mit Gemischen von Dichlormethan-Isopropylalkohol (V/V-Verhältnis von 94 : 6 bis 90 : 10) ergibt das Produkt als gelbliches Oel. Das Produkt ergibt eine Elementaranalyse, die der Theorie für die Titelverbindung $C_{48}H_{85}N_7O_{23}$ (1128,2) entspricht; Retentionszeit im Hochdruckflüssigkeitschromatogramm 4,6 Minuten (Ausgangsmaterial: 2,4 Minuten) unter den folgenden Bedingungen:

Säule: Hypersil-ODS-5µm (4,0 x 120 mm), linearer Gradient aus Lösung A, bestehend aus 2,5 millimolarem Phosphatpuffer pH 3,0, und Lösung B, bestehend aus 20 Volumenprozent 2,5 millimolarem Phosphatpuffer pH 3,0 und 80 Volumenprozent Acetonitril, bei einem Fluss von 2,3 ml/Minute.

Beispiel 5 N-(Ethoxycarbonylmethylcarbamoyl)-O-O',O"-tri-(ethoxycarbonyl)-desferrioxamin B der Formel I, worin B = $C_2H_5O$-CO-$CH_2$-NH-CO- und $A^1$ = $A^2$ = $A^3$ = $CH_3$-$CH_2$-O-CO- ist.

In analoger Weise wie im Beispiel 4, aber unter Verwendung von 40 mMol Ethyl-chlorameisensäureester als Acylierungsmittel erhält man die Titelverbindung als farbloses Oel, welches bei Elementaranalyse korrekte Werte für $C_{39}H_{67}N_7O_{17}$ (906,0) ergibt; $R_f$ = 0,40 (Chloroform: Aceton = 70 : 30), $R_f$ = 0,55 (Dichlormethan: 2-Propanol = 90 : 10), $R_f$ = 0,90 (Dichlormethan: Methanol = 80 : 20).

Beispiel 6: N,O,O',O"-Tetra-(2-ethoxycarbonyl-ethylamino-carbonyl)-desferrioxamin B

1,13 g (2 mMol) Desferrioxamin B - methansulfonat werden in 25,0 ml Pyridin vorgelegt. In die Suspension werden 2,40 ml (17 mMol) Triethylamin und anschliessend 25,6 ml (9,6 mMol) 3-Isocyanatopropionsäure-ethylester in Toluol getropft und das Reaktionsgemisch bei Raumtemperatur gerührt. Nach 4 Stunden werden dazu noch weitere 10,7 ml (4 mMol) 3-Isocyanato-propionsäure-ethylester in Toluol getropft. Nach 25 Stunden wird das überschüssige Reagenz durch Zugabe von Methanol inaktiviert. Das Reaktionsgemisch wird filtriert und bis zur Trockene eingeengt. Das Rohprodukt wird in Dichlormethan aufgenommen und mehrmals über Kieselgel chromatographiert. Das Produkt wird mit Dichlormethan/Methanol (97:3 bis 94:6) als gelbliches Oel erhalten. Das honigartige Produkt kristallisiert langsam durch. Die erhaltene Titelverbindung weist folgende Kenndaten auf: Smp. 55-60°C, $R_f$ = 0,15 (Dichlormethan:2-Propanol = 90:10), $R_f$ = 0,39 (Dichlormethan:Methanol = 90:10).

Das Ausgangsmaterial erhält man folgendermassen:

Stufe 6.1:

15,36 g (100 mMol) β-Alanin-ethylester werden in 600 ml Toluol aufgenommen und auf 90°C erwärmt. Das Edukt löst sich erst beim Aufwärmen. Dazu werden bei 90-95°C unter Argon-Atmosphäre während 10 Min. 63 ml (120 mMol) von einer 20%igen Phosgen-Toluol-Lösung zugegeben. Das Reaktionsgemisch wird bei 90°C gerührt. Nach 2 Stunden ist der grösste Teil des Eduktes umgesetzt. Das Isocyanat wird quantitativ hergestellt, indem man das Reaktionsgemisch über Nacht bei ca. 90°C rührt.

Die Reagenzienlösung, enthaltend 3-Isocyanato-propionsäure-ethylester, wird abgekühlt und weiterangesetzt.

Beispiel 7: N-(2-Ethoxycarbonyl-ethylamino-carbonyl)-desferrioxamin B

1,13 g (2 mMol) Desferrioxamin B - methansulfonat werden in 40,0 ml Pyridin vorgelegt. In die Suspension werden 0,34 ml (2,4 mMol) Triethylamin und anschliessend 3,03 ml (24 mMol) Trimethylchlorsilan zugegeben, wobei HCl-Bildung beobachtet wird. Nach 15 Min. werden während 3 Min. 6,40 ml (2,4 mMol) 3-Isocyanato-propionsäure-ethylester in Toluol (s. Stufe 6.1) zu dem silylierten Edukt getropft und das Reaktionsgemisch wird bei Raumtemperatur gerührt. Dazu werden während 29 Stunden weitere 0,34 ml (2,4 mMol) Triethylamin, 0,60 ml (4,8 mMol) Trimethylchlorsilan und 12,8 ml (4,8 mMol) 3-Isocyanatopropionsäure-ethylester in Toluol portionenweise zugegeben. Nach 48 Stunden wird das überschüssige Reagenz durch Zugabe von Methanol inaktiviert und das Reaktionsgemisch bis zur Trockene eingeengt. Der Rückstand wird in Wasser aufgenommen, wobei das Titelprodukt kristallin ausfällt. Es wird aus Dichlormethan umkristallisiert; Smp. 170–171°C.

<u>Beispiel 8:</u> N-(2-Ethoxycarbonyl-ethylamino-carbonyl)-desferrioxamin B

38 g (33 mMol) N,O,O',O"-Tetra-(2-ethoxycarbonyl-ethylamino-carbonyl)-desferrioxamin B (siehe Beispiel 6) werden in 4000 ml Acetonitril-Phosphatpuffer pH 8 (V/V = 1:2) gelöst und bei 30–37°C hydrolysieren lassen. Während mehreren Tagen verschiebt sich der Anteil an tetrasubstituiertem Ausgangsmaterial via tri- und disubstituierten Zwischenprodukten zu der gewünschten N-monosubstituierten Titelverbindung. Das Rohprodukt wird aus Wasser kristallisiert und aus Dichlormethan umkristallisiert. Das erhaltene Produkt stimmt in seinen Eigenschaften mit dem Endprodukt von Beispiel 7 überein.

<u>Beispiel 9:</u> N-(Ethoxycarbonylmethylaminocarbonyl)-desferrioxamin B

2,63 g (2, 45 mMol) N,O,O',O"-Tetra-(ethoxycarbonylmethylcarbamoyl)-desferrioxamin B werden in 340 ml Acetonitril-Phosphatpuffer pH 8 (V/V = 1:2) gelöst und bei 30°C hydrolysieren lassen. Während mehreren Tagen verschiebt sich der Anteil an tetrasubstituiertem Ausgangsmaterial via tri- und disubstituierten Zwischenprodukten zu dem gewünschten N-monosubstituierten Titelprodukt. Das Rohprodukt wird aus Wasser kristallisiert und aus Methanol-Dichlormethan umkristallisiert. Das erhaltene Titelprodukt stimmt in seinen Eigenschaften mit dem gemäss Beispiel 2 erhaltenen Produkt überein.

<u>Beispiel 10:</u> N-(2-Ethoxycarbonyl-ethylamino-carbonyl)-O,O',O"-trioctanoyl-desferrioxamin B

Ausgehend von N-(2-Ethoxycarbonyl-ethylamino-carbonyl)-desferrioxamin B erhält man die Titelverbindung analog Beispiel 3 nach Chromatographie über Kieselgel (Dichlormethan:2-Propanol = 97:3 bis 90:10, V/V) in amorpher Form.

<u>Beispiel 11:</u> N-(2-Ethoxycarbonyl-ethylamino-carbonyl)-O,O',O"-tri[2-(2-methoxy-ethoxy)-ethoxy-carbonyl]-desferrioxamin B

Ausgehend von N-(2-Ethoxycarbonyl-ethylamino-carbonyl)-desferrioxamin B erhält man die Titelverbindung analog Beispiel 4.

<u>Beispiel 12:</u> N-(2-Ethoxycarbonyl-ethylamino-carbonyl)-O,O',O"-triethoxycarbonyl-desferrioxamin B

Ausgehend von N-(2-Ethoxycarbonyl-ethylamino-carbonyl)-desferrioxamin B erhält man die Titelverbindung analog Beispiel 5.

<u>Beispiel 13:</u> N-(Ethoxycarbonylmethylaminocarbonyl)-O,O',O"-tri-(diethylaminooarbonyl)-desferrioxamin B

5,52 g (8 mMol) N-Ethoxycarbonylmethylaminocarbonyl-desferrioxamin B werden mit 100 ml Pyridin versetzt. In die erhaltene Suspension werden unter Argonatmosphäre bei 23°C 4,47 ml (32 mMol) Triethylamin und anschliessend 4,56 ml (36 mMol) Diethylcarbamoylchlorid zugetropft. Das Reaktionsgemisch wird über Nacht bei Raumtemperatur gerührt. Nach 16 Stunden ist die Lösung klar und dunkelorange. Das überschüssige Reagenz wird durch Zugabe von 100 ml Methanol inaktiviert. Das Lösungsmittel wird entfernt. Der feste Rückstand wird in 350 ml Wasser aufgenommen und mit Dichlormethan extrahiert.

Das Rohprodukt wird in Dichlormethan gelöst und über Kieselgel chromatographiert. Das Titelprodukt wird mit Dichlormethan-2-Propanol (97:3 bis 90:10) als gelbliches Öl erhalten; $R_f$ = 0,29 (Dichlormethan:2-Propanol = 90:10), $R_f$ = 0,42 (Dichlormethan:Methanol = 90:10).

<u>Beispiel 14:</u> Herstellung von 1000 Kapseln mit 260 mg des Wirkstoffes [z.B. N,O,O',O"-Tetra-(ethoxycarbonylmethylcarbamoyl)-desferrioxamin B oder eines der Endprodukte der Beispiele 3–6 und 10–13] pro Kapsel

| Zusammensetzung | |
|---|---|
| Wirkstoff | 260 g |
| Talk | 36 g |
| Weizenstärke | 24 g |
| Magnesiumstearat | 16 g |
| Laktose | 4 g |
| | 340 g |

Zubereitung

Die pulverförmigen Substanzen werden durch ein Sieb mit einer Maschenweite von 0,6 mm getrieben und gründlich gemischt. Mit je 340 mg dieser Mischung werden mit einer Kapselfüllmaschine Gelatine-Kapseln bereitet.

Beispiel 15: Herstellung von 1000 Kapseln enthaltend 105 mg des Wirkstoffes (z.B. N,O,O',O"-Tetra-(ethoxycarbonylmethylcarbamoyl)-desferrioxamin B oder eines der Endprodukte der Beispiele 3-6 und 10-13) pro Kapsel

| Zusammensetzung | |
|---|---|
| Wirkstoff | 105 g |
| Ethylcellulose | 3 g |
| Stearinsäure | 3 g |
| | 111 g |

Zubereitung

Die Ethylcellulose und die Stearinsäure werden in 120 ml Methylenchlorid gelöst, mit dem Wirkstoff versetzt, und die Masse wird durch ein Sieb von 0,6 mm Maschenweite bei einer Temperatur von ca. 40° geschlagen, wobei das Methylenchlorid verdampft. 111 mg des erhaltenen Granulates werden in Gelatine-Kapseln zu 0,5 ml mit Hilfe einer Kapsel-Abfüllmaschine abgefüllt.

Beispiel 16: N-(2-Ethoxycarbonyl-ethylamino-carbonyl)-O,O',O"-tri-(ethoxycarbonyl-methylaminocarbonyl)-desferrioxamin B

N-(2-Ethoxycarbonyl-ethylamino-carbonyl)-desferrioxamin B wird in Pyridin (mit KOH getrocknet) vorgelegt. In die Suspension werden bei Raumtemperatur unter Argonatmosphäre 3,3 Aequivalente Isocyanatoessigsäure-ethylester während 5 Min. zugetropft. Das Reaktionsgemisch wird bei Raumtemperatur gerührt. Nach 1 Stunde werden noch 2,7 Aequivalente Reagenz zugegeben.
Nach 23 Stunden werden 200 ml Methanol in die Reaktionslösung zugegeben. Nach 30minütigem Rühren werden die Lösungsmittel abdestilliert. Der feste Rückstand (am Hochvakuum getrocknet) wird in 400 ml 1 M Phosphatpuffer pH 7,4 aufgenommen und 2mal mit 350 ml Dichlormethan extrahiert. Die organischen Phasen werden durch Silikonpapier filtriert, eingeengt und am Hochvakuum getrocknet. Durch Chromatographie an Kieselgel (Dichlormethan-2-Propanol 94:6 bis 90:10) erhält man die Titelverbindung.

Beispiel 17: N-(2-Ethoxycarbonyl-ethylamino-carbonyl)-O,O',O"-tri-(diethylaminocarbonyl)-desferrioxamin B

Ausgehend von N-(2-Ethoxycarbonyl-ethylamino-carbonyl)-desferrioxamin B erhält man die Titelverbindung analog Beispiel 13.

## Patentansprüche für die benannten Vertragsstaaten: AT, BE, CH, DE, FR, GB, IT, LI, LU, NL, SE

1. Eine Verbindung der Formel I,

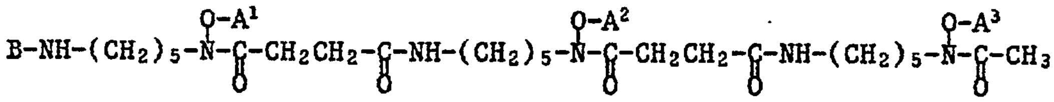

(I)

in welcher B für einen Carbamoylrest der Teilformel -CO-NH-Alk-CO-O-$R^1_a$ (II) steht, worin $R^1_a$ $C_1$-$C_4$-Alkyl oder $C_2$-$C_4$-Alkenyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und jedes der Symbole $A^1$, $A^2$ und $A^3$ unabhängig von den anderen für Wasserstoff, einen von einer Carbonsäure abgeleiteten Acylrest Ac oder einen oben definierten Carbamoylrest der Teilformel II steht, oder ein Salz einer solchen Verbindung mit salzbildenden Eigenschaften.

2. Eine Verbindung gemäss Anspruch 1, worin $A^1$, $A^2$ und $A^3$ die gleiche Bedeutung haben.

3. Eine Verbindung gemäss Anspruch 1, worin $A^1$, $A^2$ und $A^3$ je für Wasserstoff stehen.

4. Eine Verbindung gemäss Anspruch 1, worin $A^1$, $A^2$ und $A^3$ je ein und denselben Acylrest Ac oder Carbamoylrest der Teilformel II bedeuten.

5. Eine Verbindung gemäss Anspruch 2 der Formel I, in welcher jedes der Symbole $A^1$, $A^2$, $A^3$ und B für einen Rest der Teilformel -CO-NH-Alk-CO-O-$R^1_a$ (II) stehen, worin $R^1_a$ $C_1$-$C_4$-Alkyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, oder ein Salz einer solchen Verbindung mit salzbildenden Eigenschaften.

6. Eine Verbindung gemäss einem der Ansprüche 1-5, worin im Rest der Teilformel II $R^1_a$ einen linearen $C_1$-$C_4$-Alkylrest und Alk einen linearen $C_1$-$C_7$-Alkylenrest, dessen freie Valenzen von beiden endständigen C-Atomen ausgehen, darstellen.

7. Eine Verbindung gemäss einem der Ansprüche 1-5, worin im Rest der Teilformel II $R^1_a$ ein lineares $C_1$-$C_4$-Alkyl und Alk ein lineares oder einmal verzweigtes 1,1-Alkyliden darstellen.

8. Eine Verbindung gemäss einem der Ansprüche 1-5, worin der Rest der Formel II durch die Teilformel -CO-AAA-O-$R^1_a$ (IIA) dargestellt wird, worin $R^1_a$ ein lineares $C_1$-$C_4$-Alkyl und AAA einen durch die allgemeine Definition von Alk begrenzten Rest einer geläufigen $\alpha$-Aminosäure in Form eines individuellen optischen Isomeren oder eines Gemisches von mehreren Isomeren bedeuten.

9. Eine Verbindung gemäss einem der Ansprüche 1-5, worin im Rest der Teilformel II $R^1_a$ ein lineares $C_1$-$C_4$-Alkyl und Alk Methylen darstellen.

10. Eine Verbindung nach Anspruch 1, in welcher B für einen Carbamoylrest der Teilformel II steht, worin $R^1_a$ $C_1$-$C_4$-Alkyl und Alk $C_1$-$C_4$-Alkylen bedeuten, und $A^1$, $A^2$ und $A^3$ je für Wasserstoff, einen Carbamoylrest der Teilformel II, worin $R^1_a$ $C_1$-$C_4$-Alkyl und Alk $C_1$-$C_4$-Alkylen bedeuten, für Alkanoyl mit bis zu 10 C-Atomen, Niederalkoxycarbonyl, 2-(2-Methoxy-ethoxy)-ethoxy-carbonyl oder Diniederalkylamino-carbonyl stehen, wobei $A^1$, $A^2$ und $A^3$ jeweils die gleiche Bedeutung haben.

11. Eine Verbindung nach Anspruch 1, in welcher B Ethoxycarbonylmethylaminocarbonyl oder 2-Ethoxycarbonyl-ethylamino-carbonyl und $A^1$, $A^2$ und $A^3$ je Ethoxycarbonylmethylaminocarbonyl, 2-Ethoxy carbonylethylamino-carbonyl, Wasserstoff, n-Octanoyl, Ethoxycarbonyl, 2-(2-Methoxy-ethoxy)-ethoxy-carbonyl oder Diethylaminocarbonyl bedeuten, wobei $A^1$, $A^2$ und $A^3$ jeweils die gleiche Bedeutung haben.

12. Eine Verbindung nach Anspruch 1, 10 oder 11, worin $A^1$, $A^2$ und $A^3$ je Ethoxycarbonylmethylaminocarbonyl, 2-Ethoxycarbonyl-ethylamino-carbonyl, n-Octanoyl, Ethoxycarbonyl oder 2-(2-Methoxy-ethoxy)-ethoxy-carbonyl bedeuten, wobei $A^1$, $A^2$ und $A^3$ jeweils die gleiche Bedeutung haben.

13. Eine Verbindung nach Anspruch 1 oder 10, worin $A^1$, $A^2$ und $A^3$ je Diniederalkylaminocarbonyl bedeuten.

14. N,O,O',O''-Tetra-(ethoxycarbonylmethylcarbamoyl)-desferrioxamin B und N,O,O',O''-Tetra-(2-ethoxycarbonyl-ethylamino-carbonyl) desferrioxamin B nach Anspruch 1.

15. N-(Ethoxycarbonylmethylcarbamoyl)-O,O',O''-tri-octanoyl-desferrioxamin B und N-(2-Ethoxycarbonyl-ethylamino-carbonyl)-O,O',O''trioctanoyl-desferrioxamin B nach Anspruch 1.

16. N-( Ethoxycarbonylmethylcarbamoyl) -O,O',O''-tri-ethoxycarbonyldesferrioxamin B, N-( 2-Ethoxycarbonyl-ethylamino-carbonyl) -O,O',O''tri-(ethoxycarbonyl)-desferrioxamin B und N-(2-Ethoxycarbonylethylamino-carbonyl)-O,O',O''-tri-(ethoxycarbonylmethylaminocarbonyl)-desferrioxamin B nach

Anspruch 1.

17. N-(Ethoxycarbonylmethylcarbamoyl)-O,O',O''-tri-[2-(2-methoxyethoxy)-ethoxycarbonyl ] -desferrioxamin B und N-( 2-Ethoxycarbonylethylamino-carbonyl)-O,O',O''-tri-[2-(2-methoxy-ethoxy)-ethoxycarbonyl] -desferrioxamin B nach Anspruch 1.

18. N-Ethoxycarbonylmethylaminocarbonyl-O,O',O''-tri-( diethylaminocarbonyl)-desferrioxamin B nach Anspruch 1.

19. Verfahren zur Herstellung einer Verbindung der Formel I nach Anspruch 1, dadurch gekennzeichnet, dass man

a) ein Derivat von Desferrioxamin B der Formel IV,

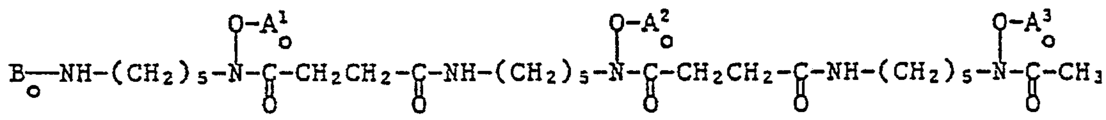

(IV)

worin $B_o$ Wasserstoff oder eine organische Silylgruppe Sil ist und jedes der Symbole $A^1_o$, $A^2_o$ und $A^3_o$ unabhängig von den anderen Wasserstoff, eine organische Silylgruppe Sil oder einen oben definierten Acylrest Ac bedeutet, mit einem Isocyanatocarbonsäureester der Formel III,

$$O{=}C{=}N{-}Alk{-}CO{-}O{-}R^1_a \qquad (III)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, oder

b) ein Derivat von Desferrioxamin B der Formel V,

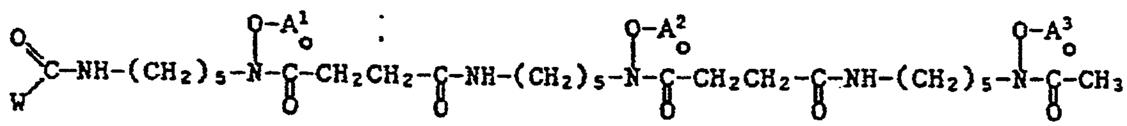

(V)

in welcher W Chlor oder 1-Imidazolyl ist, und worin jedes der Symbole $A^1_o$, $A^2_o$ und $A^3_o$ unabhängig von den anderen Wasserstoff, eine organische Silylgruppe Sil oder einen oben definierten Acylrest Ac bedeutet, mit einem Aminosäureester der Formel

$$H_2N{-}Alk{-}CO{-}O{-}R^1_a \qquad (VI)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, und gegebenenfalls vorhandene Silylgruppen und/oder Aminoschutzgruppen unter Freisetzung der entsprechenden Hydroxyl- bzw. Aminogruppen abspaltet und, wenn erwünscht, eine erhaltene Verbindung der Formel I, worin mindestens eines der Symbole $A^1$, $A^2$ und $A^3$ Wasserstoff ist, zu einer Verbindung der Formel I, worin die entsprechenden Symbole für Ac oder einen Carbamoylrest der Teilformel II stehen, acyliert und/oder, wenn erwünscht, eine erhaltene freie Verbindung der Formel I mit salzbildenden Eigenschaften in ein Salz umwandelt und/oder eine Verbindung der Formel I aus einem solchen Salz freisetzt.

20. Eine Verbindung gemäss einem der Ansprüche 1–18 zur Anwendung in einem Verfahren zur therapeutischen Behandlung des menschlichen oder tierischen Körpers.

21. Pharmazeutische Zusammensetzungen, enthaltend neben pharmazeutischem Trägermaterial als Wirkstoff eine Verbindung der Formel I nach einem der Ansprüche 1–18 oder ein pharmazeutisch verwendbares Salz einer solchen Verbindung mit salzbildenden Eigenschaften.

22. Pharmazeutische Zusammensetzungen gemäss Anspruch 21 in Form von Dragees, Tabletten oder Kapseln zur oralen Verabreichung.

23. Verwendung einer Verbindung gemäss einem der Ansprüche 1–18 zur Herstellung von pharmazeutischen Zusammensetzungen, die für die Anwendung zur Behandlung von krankhaften Zuständen in Warmblütern einschliesslich des Menschen, welche mit einem Überschuss an Eisen(III) oder Aluminium im Körper zusammenhängen oder durch Eisen(III)-abhängige pathogene Organismen verursacht werden, bestimmt sind.

**Patentansprüche für die benannten Vertragsstaaten: ES, GR**

1. Verfahren zur Herstellung einer Verbindung der Formel I,

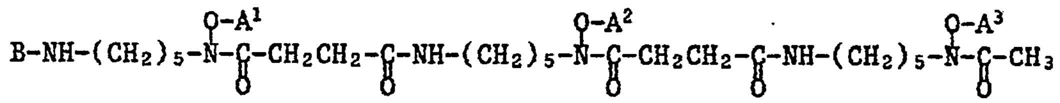

(I)

in welcher B für einen Carbamoylrest der Teilformel -CO-NH-Alk-CO-O-$R^1_a$ (II) steht, worin $R^1_a$ ein $C_1$-$C_4$-Alkyl oder $C_2$-$C_4$-Alkenyl und Alk ein unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, und jedes der Symbole $A^1$, $A^2$ und $A^3$ unabhängig von den anderen für Wasserstoff, einen von einer Carbonsäure abgeleiteten Acylrest Ac oder einen oben definierten Carbamoylrest der Teilformel II steht oder eines Salzes einer solchen Verbindung mit salzbildenden Eigenschaften, dadurch gekennzeichnet, dass man
a) ein Derivat von Desferrioxamin B der Formel IV,

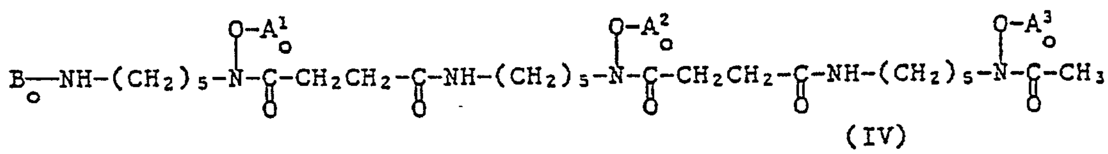

(IV)

worin $B_o$ Wasserstoff oder eine organische Silylgruppe Sil ist und jedes der Symbole $A^1_o$, $A^2_o$ und $A^3_o$ unabhängig von den anderen Wasserstoff, eine organische Silylgruppe Sil oder einen oben definierten Acylrest Ac bedeutet, mit einem Isocyanatocarbonsäureester der Formel III,

$$O{=}C{=}N\text{-}Alk\text{-}CO\text{-}O\text{-}R^1_a \qquad (III)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, oder
b) ein Derivat von Desferrioxamin B der Formel V,

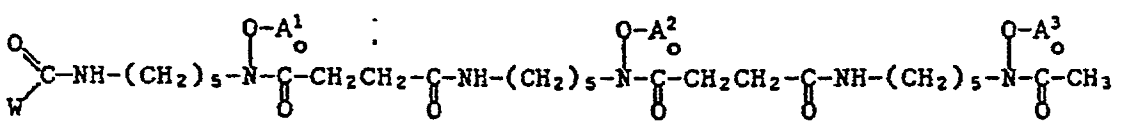

(V)

in welcher W Chlor oder 1-Imidazolyl ist, und worin jedes der Symbole $A^1_O$, $A^2_O$ und $A^3_O$ unabhängig von den anderen Wasserstoff, eine organische Silylgruppe Sil oder einen oben definierten Acylrest Ac bedeutet, mit einem Aminosäureester der Formel

$$H_2N\text{-}Alk\text{-}CO\text{-}O\text{-}R^1_a \qquad (VI)$$

worin Alk und $R^1_a$ die oben genannten Bedeutungen haben und in welchem eine im Rest Alk gegebenenfalls anwesende zusätzliche Aminogruppe in geschützter Form vorliegt, umsetzt, und gegebenenfalls vorhandene Silylgruppen und/oder Aminoschutzgruppen unter Freisetzung der entsprechenden Hydroxyl- bzw. Aminogruppen abspaltet und, wenn erwünscht, eine erhaltene Verbindung der Formel I, worin mindestens eines der Symbole $A^1$, $A^2$ und $A^3$ Wasserstoff ist, zu einer Verbindung der Formel I, worin die entsprechenden Symbole für Ac oder einen Carbamoylrest der Teilformel II stehen, acyliert und/oder, wenn erwünscht, eine erhaltene freie Verbindung der Formel I mit salzbildenden Eigenschaften in ein Salz umwandelt und/oder eine Verbindung der Formel I aus einem solchen Salz freisetzt.

2. Verfahren gemäss Anspruch 1 zur Herstellung einer Verbindung der Formel I, in welcher jedes der Symbole $A^1$, $A^2$, $A^3$ und B für einen Rest der Teilformel -CO-NH-Alk-CO-O-$R^1_a$ (II) stehen, worin $R^1_a$ $C_1$-$C_4$-Alkyl und Alk unsubstituiertes oder durch Hydroxyl, $C_1$-$C_4$-Alkanoyloxy, Amino, $C_1$-$C_4$-Alkoxycarbony, Carbamoyl, Phenyl, Hydroxyphenyl, Methoxyphenyl oder Indolyl substituiertes $C_1$-$C_7$-Alkylen bedeuten, oder eines Salzes einer solchen Verbindung mit salzbildenden Eigenschaften, dadurch gekennzeichnet, dass man Desferrioxamin B oder ein Säureadditionsalz davon mit einem Isocyanatocarbonsäureester der Formel

$$O{=}C{=}N\text{-}Alk\text{-}CO\text{-}O\text{-}R^1_a \qquad (III)$$

worin Alk und $R^1_a$ die obgenannten Bedeutungen haben, und in welchem eine im Rest Alk gegebenenfalls anwesende Aminogruppe in geschützter Form vorliegt, umsetzt und eine gegebenenfalls vorhandene Aminogruppe durch Abspalten der Aminoschutzgruppe freisetzt, und gewünschtenfalls eine freie Verbindung der Formel I mit salzbildenden Eigenschaften in ein Salz umwandelt und/oder eine Verbindung der Formel I aus einem solchen Salz freisetzt.

3. Verfahren gemäss Anspruch 1, dadurch gekennzeichnet, dass man zur Herstellung von Verbindungen der Formel I, worin B eine andere Bedeutung als $A^1$, $A^2$ und $A^3$ hat, von einer Verbindung der Formel IV ausgeht, worin $B_o$ sowie mindestens eines der Symbole $A^1_o$, $A^2_o$ und $A^3_o$, für eine organische Silylgruppe (Sil) der Formel

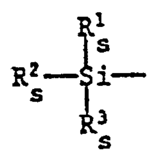 (Sil),

stehen, in welcher $R^1_s$ und $R^2_s$ unabhängig voneinander je ein unsubstituiertes $C_1$-$C_8$-Hydrocarbyl und $R^3_s$ ein unsubstituiertes $C_1$-$C_8$-Hydrocarbyl oder Chlor bedeutet.

4. Verfahren gemäss Anspruch 1, dadurch gekennzeichnet, dass man zur Herstellung von Verbindungen der Formel I, worin $A^1$, $A^2$ und $A^3$ für Wasserstoff stehen, von einer Verbindung der Formel IV ausgeht, worin $B_o$, $A^1_o$, $A^2_o$ und $A^3_o$ alle für die im Anspruch 3 definierte Gruppe Sil stehen.

5. Verfahren gemäss Anspruch 3 oder 4, dadurch gekennzeichnet, dass man den in diesen Ansprüchen definierten Ausgangsstoff der Formel IV mit dem in Anspruch 1 definierten Reagens der Formel III umsetzt und anschliessend die Silylgruppen solvolytisch abspaltet.

6. Verfahren gemäss Anspruch 5, dadurch gekennzeichnet, dass man den Ausgangsstoff der Formel IV in situ dadurch herstellt, dass man Desferrioxamin B oder ein Salz davon mit einem Silylierungsreagens der Formel Sil-Hal, worin Hal für Brom oder Chlor steht und Sil die im Anspruch 3 angegebene Bedeutung hat, in einer heterocyclischen aprotischen Base vom Typ Pyridin umsetzt und anschliessend in demselben Reaktionsmedium die Umsetzung mit dem Reagens der Formel III und die Abspaltung von Silylgruppen vornimmt.

7. Verfahren gemäss Anspruch 1, dadurch gekennzeichnet, dass man die Ausgangsstoffe so wählt, dass man eine Verbindung der Formel I herstellt,worin $A^1$, $A^2$ und $A^3$ die gleiche Bedeutung haben.

8. Verfahren gemäss Anspruch 1, dadurch gekennzeichnet, dass man die Ausgangsstoffe so wählt, dass man eine Verbindung der Formel I herstellt, worin $A^1$, $A^2$ und $A^3$ je für Wasserstoff stehen.

9. Verfahren gemäss Anspruch 1, dadurch gekennzeichnet, dass man die Ausgangsstoffe so wählt, dass man eine Verbindung der Formel I herstellt, worin $A^1$, $A^2$ und $A^3$ je ein und denselben Acylrest Ac oder Carbamoylrest der Teilformel II bedeuten.

10. Verfahren gemäss Anspruch 1, dadurch gekennzeichnet, dass man die Ausgangsstoffe so wählt, dass man eine Verbindung der Formel I herstellt, in welcher jedes der Symbole $A^1$, $A^2$, $A^3$ und B für einen Rest der Teilformel -CO-NH-Alk-CO-O-$R^1_a$ (II) stehen, worin $R^1_a$ $C_1$-$C_4$-Alkyl und Alk unsubstituiertes oder durch Hydroxyl, $C_1$-$C_4$-Alkanoyloxy, Amino, $C_1$-$C_4$-Alkoxycarbonyl, Carbamoyl, Phenyl, Hydroxyphenyl, Methoxyphenyl oder Indolyl substituiertes $C_1$-$C_7$-Alkylen bedeuten, oder ein Salz einer solchen Verbindung mit salzbildenden Eigenschaften.

11. Verfahren gemäss einem der Ansprüche 1, 2 und 7-10, dadurch gekennzeichnet, dass man die Ausgangsstoffe so wählt, dass man eine Verbindung der Formel I herstellt, worin im Rest der Teilformel II $R^1_a$ einen linearen $C_1$-$C_4$-Alkylrest und Alk einen linearen $C_1$-$C_7$-Alkylenrest, dessen freie Valenzen von beiden endständigen C-Atomen ausgehen, darstellen.

12. Verfahren gemäss einem der Ansprüche 1, 2 und 7-10, dadurch gekennzeichnet, dass man die Ausgangsstoffe so wählt, dass man eine Verbindung der Formel I herstellt, worin im Rest der Teilformel II $R^1_a$ ein lineares $C_1$-$C_4$-Alkyl und Alk ein lineares oder einmal verzweigtes 1,1-Alkyliden darstellen.

13. Verfahren gemäss einem der Ansprüche 1, 2 und 7-10, dadurch gekennzeichnet, dass man die Ausgangsstoffe so wählt, dass man eine Verbindung der Formel I herstellt, worin der Rest der Formel II durch die Teilformel -CO-AAA-O-$R^1{}_a$ (IIA) dargestellt wird, worin $R^1{}_a$ ein lineares $C_1$-$C_4$-Alkyl und AAA einen durch die allgemeine Definition von Alk begrenzten Rest einer geläufigen $\alpha$-Aminosäure in Form eines individuellen optischen Isomeren oder eines Gemisches von mehreren Isomeren bedeuten.

14. Verfahren gemäss einem der Ansprüche 1, 2 und 7-10, dadurch gekennzeichnet, dass man die Ausgangsstoffe so wählt, dass man die Verbindung der Formel I herstellt, worin im Rest der Teilformel II $R^1{}_a$ ein lineares $C_1$-$C_4$-Alkyl und Alk Methylen darstellen.

15. Verfahren nach Anspruch 1, dadurch gekennzeichnet, dass man die Ausgangsstoffe so wählt, dass man eine Verbindung der Formel I herstellt, in welcher B für einen Carbamoylrest der Teilformel II steht, worin $R^1{}_a$ $C_1$-$C_4$-Alkyl und Alk $C_1$-$C_4$-Alkylen bedeuten, und $A^1$, $A^2$ und $A^3$ je für Wasserstoff, einen Carbamoylrest der Teilformel II, worin $R^1{}_a$ $C_1$-$C_4$-Alkyl und Alk $C_1$-$C_4$-Alkylen bedeuten, für Alkanoyl mit bis zu 10 C-Atomen, Niederalkoxycarbonyl, 2-(2-Methoxy-ethoxy)-ethoxy-carbonyl oder Diniederalkylamino-carbonyl stehen, wobei $A^1$, $A^2$ und $A^3$ jeweils die gleiche Bedeutung haben.

16. Verfahren nach Anspruch 1, dadurch gekennzeichnet, dass man die Ausgangsstoffe so wählt, dass man eine Verbindung der Formel I herstellt, in welcher B Ethoxycarbonylmethylaminocarbonyl oder 2-Ethoxycarbonyl-ethylamino-carbonyl und $A^1$, $A^2$ und $A^3$ je Ethoxycarbonylmethylaminocarbonyl, 2-Ethoxycarbonylethylamino-carbonyl, Wasserstoff, n-Octanoyl, Ethoxycarbonyl, 2-(2-Methoxy-ethoxy)-ethoxy-carbonyl oder Diethylaminocarbonyl bedeuten, wobei $A^1$, $A^2$ und $A^3$ jeweils die gleiche Bedeutung haben.

17. Verfahren nach Anspruch 1, 15 oder 16, dadurch gekennzeichnet, dass man die Ausgangsstoffe so wählt, dass man eine Verbindung der Formel I herstellt, worin $A^1$, $A^2$ und $A^3$ je Ethoxycarbonylmethylaminocarbonyl, 2-Ethoxycarbonyl-ethylamino-carbonyl, n-Octanoyl, Ethoxycarbonyl oder 2-(2-Methoxy-ethoxy)-ethoxy-carbonyl bedeuten, wobei $A^1$, $A^2$ und $A^3$ jeweils die gleiche Bedeutung haben.

18. Verfahren nach Anspruch 1, 15 oder 16, dadurch gekennzeichnet, dass man die Ausgangsstoffe so wählt, dass man eine Verbindung der Formel I herstellt, worin $A^1$, $A^2$ und $A^3$ je Diniederalkylaminocarbonyl bedeuten.

19. Verfahren nach Anspruch 1 oder 2, dadurch gekennzeichnet, dass man die Ausgangsstoffe so wählt, dass man N,O,O',O"-Tetra-(ethoxycarbonylmethylcarbamoyl)-desferrioxamin B oder N,O,O', O"-Tetra-(2ethoxycarbonylethylamino-carbonyl)-desferrioxamin B herstellt.

20. Verfahren nach Anspruch 1 oder 3, dadurch gekennzeichnet, dass man die Ausgangsstoffe so wählt, dass man N-(Ethoxycarbonylmethylcarbamoyl)-O,O',O"-tri-octanoyl-desferrioxamin B oder N-(2-Ethoxycarbonyl-ethylamino-carbonyl) -O,O',O"-trioctanoyldesferrioxamin B herstellt.

21. Verfahren nach Anspruch 1 oder 3, dadurch gekennzeichnet, dass man die Ausgangsstoffe so wählt, dass man N-(Ethoxycarbonylmethylcarbamoyl)-O,O',O"-tri-ethoxycarbonyl-desferrioxamin B, N-(2-Ethoxy carbonyl-ethylamino-carbonyl)-O,O',O"-tri-(ethoxycarbonyl)-desferrioxamin B oder N-(2-Ethoxycarbonyl-ethylamino-carbonyl)-O,O',O"tri-(ethoxycarbonylmethylaminocarbonyl)-desferrioxamin B herstellt.

22. Verfahren nach Anspruch 1 oder 3, dadurch gekennzeichnet, dass man die Ausgangsstoffe so wählt, dass man N-(Ethoxycarbonylmethylcarbamoyl)-O,O',O"-tri-[2-(2-methoxyethoxy)-ethoxycarbonyl]-desferrioxamin B oder N-(2-Ethoxycarbonyl-ethylamino-carbonyl)-O, O',O"-tri-[2-(2-methoxy-ethoxy)-ethoxy-carbonyl]-desferrioxamin B herstellt.

23. Verfahren nach Anspruch 1 oder 3, dadurch gekennzeichnet, dass man die Ausgangsstoffe so wählt, dass man N-Ethoxycarbonylmethylaminocarbonyl-O,O',O"-tri-(diethylaminocarbonyl)-desferrioxamin B herstellt.

**Claims for the Contracting States: AT, BE, CH, DE, FR, GB, IT, LI, LU, NL, SE**

1. A compound of the formula I

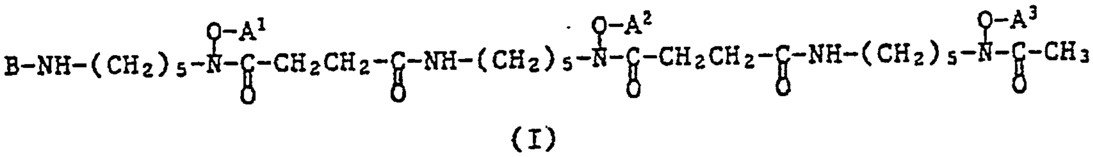

(I)

in which B represents a carbamoyl radical of the partial formula -CO-NH-Alk-CO-O-$R^1{}_a$ (II) in which $R^1{}_a$ represents $C_1$-$C_4$-alkyl or $C_2$-$C_4$-alkenyl and Alk represents $C_1$-$C_7$-alkylene that is unsubstituted or substituted by hydroxy, $C_1$-$C_4$-alkanoyloxy, amino, $C_1$-$C_4$-alkoxycarbonyl, carbamoyl, phenyl, hydroxyphenyl, methoxyphenyl or by indolyl, and each of the symbols $A^1$, $A^2$ and $A^3$, independently of the others, represents hydrogen, an acyl radical Ac derived from a carboxylic acid, or an above-defined carbamoyl radical of the partial formula II, or a salt of such a compound having salt-forming properties.

2. A compound according to claim 1 in which $A^1$, $A^2$ and $A^3$ have the same meaning.

3. A compound according to claim 1 in which each of $A^1$, $A^2$ and $A^3$ represents hydrogen.

4. A compound according to claim 1 in which each of $A^1$, $A^2$ and $A^3$ represents one and the same acyl radical Ac or carbamoyl radical of the partial formula II.

5. A compound according to claim 2 of the formula I in which each of the symbols $A^1$, $A^2$, $A^3$ and B represents a radical of the partial formula -CO-NH-Alk-CO-O-$R^1_a$ (II) in which $R^1_a$ represents $C_1$-$C_4$-alkyl and Alk represents $C_1$-$C_7$-alkylene that is unsubstituted or substituted by hydroxy, $C_1$-$C_4$-alkanoyloxy, amino, $C_1$-$C_4$-alkoxycarbonyl, carbamoyl, phenyl, hydroxyphenyl, methoxyphenyl or by indolyl, or a salt of such a compound having salt-forming properties.

6. A compound according to any one of claims 1 to 5 in which in the radical of the partial formula II $R^1_a$ represents a linear $C_1$-$C_4$-alkyl radical and Alk represents a linear $C_1$-$C_7$-alkylene radical, the free valencies of which originate from the two terminal carbon atoms.

7. A compound according to any one of claims 1 to 5 in which in the radical of the partial formula II $R^1_a$ represents a linear $C_1$-$C_4$-alkyl radical and Alk represents a linear 1,1-alkylidene radical or a 1,1-alkylidene radical branched once.

8. A compound according to any one of claims 1 to 5 in which the radical of the formula II is represented by the partial formula -CO-AAA-O-$R^1_a$ (IIA) in which $R^1_a$ represents a linear $C_1$-$C_4$-alkyl radical and AAA represents a radical of a common $\alpha$-amino acid limited by the general definition of Alk and in the form of an individual optical isomer or a mixture of several isomers.

9. A compound according to any one of claims 1 to 5 in which in the radical of the partial formula II $R^1_a$ represents a linear $C_1$-$C_4$-alkyl radical and Alk represents methylene.

10. A compound according to claim 1 in which B represents a carbamoyl radical of the partial formula II in which $R^1_a$ represents $C_1$-$C_4$-alkyl and Alk represents $C_1$-$C_4$alkylene and each of $A^1$, $A^2$ and $A^3$ represents hydrogen, alkanoyl having up to 10 carbon atoms, lower alkoxycarbonyl, 2--(2-methoxyethoxy)-ethoxycarbonyl, di-lower alkylaminocarbonyl or a carbamoyl radical of the partial formula II in which $R^1_a$ represents $C_1$-$C_4$-alkyl and Alk represents $C_1$-$C_4$-alkylene, $A^1$, $A^2$ and $A^3$ all having the same meaning.

11. A compound according to claim 1 in which B represents ethoxycarbonylmethylaminocarbonyl or 2-ethoxycarbonylethylaminocarbonyl and each of $A^1$, $A^2$ and $A^3$ represents ethoxycarbonylmethylaminocarbonyl, 2-ethoxycarbonylethylaminocarbonyl, hydrogen, n-octanoyl, ethoxycarbonyl, 2-(2-methoxyethoxy)-ethoxycarbonyl or diethylaminocarbonyl, $A^1$, $A^2$ and $A^3$ all having the same meaning.

12. A compound according to claim 1, 10 or 11 in which each of $A^1$, $A^2$ and $A^3$ represents ethoxycarbonylmethylaminocarbonyl, 2-ethoxycarbonylethylaminocarbonyl, noctanoyl, ethoxycarbonyl or 2-(2-methoxyethoxy)-ethoxycarbonyl, $A^1$, $A^2$ and $A^3$ all having the same meaning.

13. A compound according to claim 1 or 10 in which each of $A^1$, $A^2$ and $A^3$ represents di-lower alkylaminocarbonyl.

14. N,O,O',O''-tetra-(ethoxycarbonylmethylcarbamoyl)desferrioxamine B and N, O, O', O''-tetra-(2-ethoxycarbonylethylaminocarbonyl)-desferrioxamine B according to claim 1.

15. N-(ethoxycarbonylmethylcarbamoyl)-O,O',O''-trioctanoyl-desferrioxamine B and N-2-ethoxycarbonylethylaminocarbonyl)-O,O',O''-trioctanoyl-desferrioxamine B according to claim 1.

16. N-(ethoxycarbonylmethylcarbamoyl)-O,O',O''-triethoxycarbonyl-desferrioxamine B, N-(2-ethoxycarbonylethyl aminocarbonyl)-O,O',O''-tri-(ethoxycarbonyl)-desferrioxamine B and N-(2-ethoxycarbonylethylaminocarbonyl)O,O',O''-tri-(ethoxycarbonylmethylaminocarbonyl)-desferrioxamine B according to claim 1.

17. N-(ethoxycarbonylmethylcarbamoyl)-O,O',O''-tri-[2-(2methoxyethoxy)-ethoxycarbonyl]-desferrioxamine B and N-(2-ethoxycarbonylethylaminocarbonyl)-O,O',O''-tri-[2-(2methoxyethoxy)-ethoxycarbonyl]-desferrioxamine B according to claim 1.

18. N-ethoxycarbonylmethylaminocarbonyl-O,O',O''-tri(diethylaminocarbonyl)-desferrioxamine B according to claim 1.

19. A process for the manufacture of a compound of the formula I according to claim 1, characterised in that

a) a derivative of desferrioxamine B of the formula IV

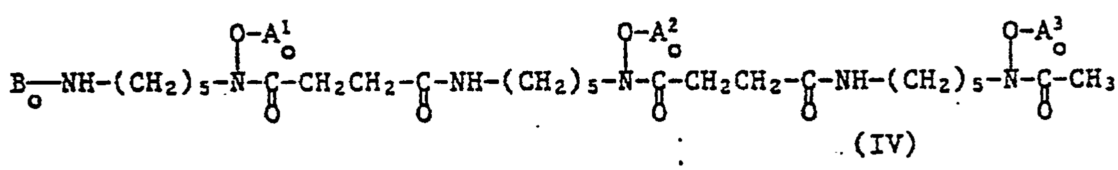

(IV)

in which $B_O$ represents hydrogen or an organic silyl group Sil and each of the symbols $A_O^1$, $A_O^2$ and $A_O^3$, independently of the others, represents hydrogen, an organic silyl group Sil or an above-defined acyl radical Ac, is reacted with an isocyanatocarboxylic acid ester of the formula III

$$O=C=N-Alk-CO-O-R^1_a \qquad (III)$$

in which Alk and $R^1_a$ have the meanings given above and in which an additional amino group that may be present in the radical Alk is in protected form, or

b) a derivative of desferrioxamine B of the formula V

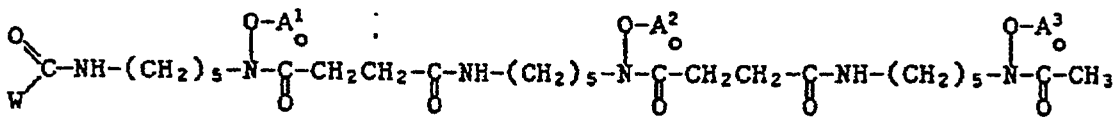

(V)

in which W is chlorine or l-imidazolyl, and in which each of the symbols $A^1_O$, $A^2_O$ and $A^1_O$, independently of the others, represents hydrogen, an organic silyl group Sil or an above-defined acyl radical Ac, is reacted with an amino acid ester of the formula

$$H_2N-Alk-CO-O-R^1_a \qquad (VI)$$

in which Alk and $R^1_a$ have the meanings given above and in which an additional amino group that may be present in the radical Alk is in protected form, and, if desired, any silyl groups and/or amino-protecting groups that may be present are removed to free the corresponding hydroxy and amino groups, respectively, and, if desired, a resulting compound of the formula I in which at least one of the symbols $A^1$, $A^2$ and $A^3$ is hydrogen is acylated to a compound of the formula I in which the corresponding symbols represent Ac or a carbamoyl radical of the partial formula II and/or, if desired, a resulting free compound of the formula I having salt-forming properties is converted into a salt and/or a compound of the formula I is freed from such a salt.

20. A compound according to any one of claims 1 to 18 for use in a method for the therapeutic treatment of the human or animal body.

21. Pharmaceutical compositions containing as active ingredient a compound of the formula I according to any one of claims 1 to 18 or a pharmaceutically acceptable salt of such a compound having salt-forming properties, together with a pharmaceutical carrier.

22. Pharmaceutical compositions according to claim 21 in the form of dragees, tablets or capsules for oral administration.

23. Use of a compound according to any one of claims 1 to 18 for the manufacture of pharmaceutical compositions for the treatment of pathological conditions in warm-blooded animals, including humans, that are associated with an excess of iron(III) or aluminium in the body or are caused by iron(III)-dependent pathogenic organisms.

**Claims for the Contracting States: ES, GR**

1. A process for the manufacture of a compound of the formula I

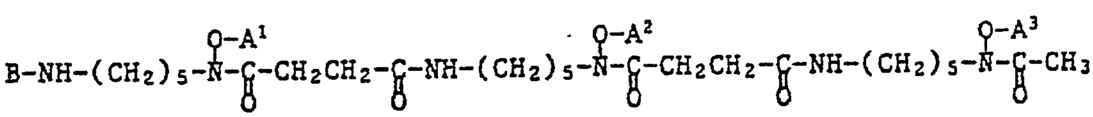

(I)

in which B represents a carbamoyl radical of the partial formula -CO-NH-Alk-CO-O-$R^1_a$ (II) in which $R^1_a$ represents $C_1$-$C_4$-alkyl or $C_2$-$C_4$-alkenyl and Alk represents $C_1$-$C_7$-alkylene that is unsubstituted or substituted by hydroxy, $C_1$-$C_4$-alkanoyloxy, amino, $C_1$-$C_4$-alkoxycarbonyl, carbamoyl, phenyl, hydroxyphenyl, methoxyphenyl or by indolyl, and each of the symbols $A^1$, $A^2$ and $A^3$, independently of the others, represents hydrogen, an acyl radical Ac derived from a carboxylic acid, or an above-defined carbamoyl radical of the partial formula II, or a salt of such a compound having salt-forming properties, characterised in that

a) a derivative of desferrioxamine B of the formula IV

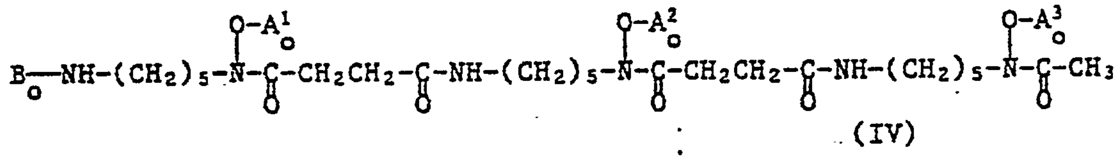

(IV)

in which $B_O$ represents hydrogen or an organic silyl group Sil and each of the symbols $A^1_O$, $A^2_O$ and $A^3_O$, Independently of the others, represents hydrogen, an organic silyl group Sil or an above-defined acyl radical Ac, is reacted with an isocyanatocarboxylic acid ester of the formula III

$$O=C=N-Alk-CO-O-R^1_a \qquad (III)$$

in which Alk and $R^1_a$ have the meanings given above and in which an additional amino group that may be present in the radical Alk is in protected form, or

b) a derivative of desferrioxamine B of the formula V

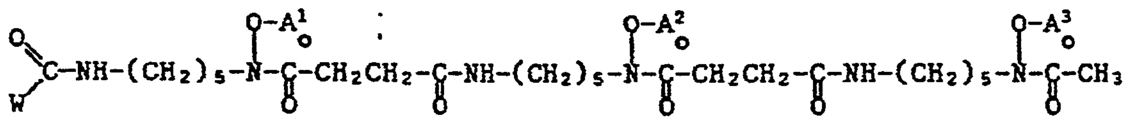

(V)

in which W is chlorine or 1-imidazolyl, and in which each of the symbols $A^1_O$, $A^2_O$ and $A^3_O$, independently of the others, represents hydrogen, an organic silyl group Sil or an above-defined acyl radical Ac, is reacted with an amino acid ester of the formula

$$H_2N-Alk-CO-O-R^1_a \qquad (VI)$$

in which Alk and $R^1_a$ have the meanings given above and in which an additional amino group that may be present in the radical Alk is in protected form, and, if desired, any silyl groups and/or amino-protecting groups that may be present are removed to free the corresponding hydroxy and amino groups, respectively, and, if desired, a resulting compound of the formula I in which at least one of the symbols $A^1$, $A^2$ and $A^3$ is hydrogen is acylated to a compound of the formula I in which the corresponding symbols represent Ac or a carbamoyl radical of the partial formula II and/or, if desired, a resulting free compound of the formula I having salt-forming properties is converted into a salt and/or a compound of the formula I is freed from such a salt.

2. A process according to claim 1 for the manufacture of a compound of the formula I in which each of the symbols $A^1$, $A^2$, $A^3$ and B represents a radical of the partial formula $-CO-NH-Alk-CO-O-R^1_a$ (II) in which $R^1_a$ represents $C_1$-$C_4$-alkyl and Alk represents $C_1$-$C_7$-alkylene that is unsubstituted or substituted by hydroxy, $C_1$-$C_4$-alkanoyloxy, amino, $C_1$-$C_4$-alkoxycarbonyl, carbamoyl, phenyl, hydroxyphenyl, methoxyphenyl or by indolyl, or a salt of such a compound having salt-forming properties, characterised in that desferrioxamine B or an acid addition salt thereof is reacted with an isocyanatocarboxylic acid ester of the formula

$$O=C=N-Alk-CO-O-R^1_a \qquad (III)$$

in which Alk and $R^1_a$ have the meanings given above and in which an amino group that may be present in the radical Alk is in protected form, and an amino group that may be present is freed by removing the amino-protecting group and, if desired, a free compound of the formula I having salt-forming properties is converted into a salt and/or a compound of the formula I is freed from such a salt.

3. A process according to claim 1, characterised in that to manufacture compounds of the formula I in which B has a meaning other than $A^1$, $A^2$ and $A^3$, a compound of the formula IV is used as starting material in which $B_O$ and at least one of the symbols $A^1_O$, $A^2_O$ and $A^3_O$ represents an organic silyl group (Sil) of the formula

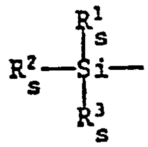 (Sil),

in which each of $R^1_s$ and $R^2_s$, independently of the other, represents an unsubstituted $C_1$-$C_8$-hydrocarbyl and $R^3_s$ represents an unsubstituted $C_1$-$C_8$-hydrocarbyl or chlorine.

4. A process according to claim 1, characterised in that to manufacture compounds of the formula I in which $A^1$, $A^2$ and $A^3$ represent hydrogen, a compound of the formula IV is used as starting material in which $B_O$, $A^1_O$, $A^2_O$ and $A^3_O$ all represent the group Sil defined in claim 3.

5. A process according to claim 3 or 4, characterised in that the starting material of the formula IV defined in those claims is reacted with the reagent of the formula III defined in claim 1 and then the silyl groups are removed by solvolysis.

6. A process according to claim 5, characterised in that the starting material of the formula IV is manufactured in situ as follows: desferrioxamine B or a salt thereof is reacted with a silylation reagent of the formula Sil-Hal, in which Hal represents bromine or chlorine and Sil has the meaning given in claim 3, in a heterocyclic aprotic base of the pyridine type, and then the reaction with the reagent of the formula III and the removal of silyl groups are carried out in the same reaction medium.

7. A process according to claim 1, characterised in that the starting materials are so selected that a compound of formula I is manufactured in which $A^1$, $A^2$ and $A^3$ have the same meaning.

8. A process according to claim 1, characterised in that the starting materials are so selected that a compound of formula I is manufactured in which each of $A^1$, $A^2$ and $A^3$ represents hydrogen.

9. A process according to claim 1, characterised in that the starting materials are so selected that a compound of formula I is manufactured in which each of $A^1$, $A^2$ and $A^3$ represents one and the same acyl radical Ac or carbamoyl radical of the partial formula II.

10. A process according to claim 1, characterised in that the starting materials are so selected that a compound of formula I in which each of the symbols $A^1$, $A^2$, $A^3$ and B represents a radical of the partial formula -CO-NH-Alk-CO-O-$R^1_a$ (II) in which $R^1_a$ represents $C_1$-$C_4$-alkyl and Alk represents $C_1$-$C_7$-alkylene that is unsubstituted or substituted by hydroxy, $C_1$-$C_4$-alkanoyloxy, amino, $C_1$-$C_4$-alkoxycarbonyl, carbamoyl, phenyl, hydroxyphenyl, methoxyphenyl or by indolyl, or a salt of such a compound having salt-forming properties is manufactured.

11. A process according to any one of claims 1, 2 and 7–10, characterised in that the starting materials are so selected that a compound of formula I is manufactured in which in the radical of the partial formula II $R^1_a$ represents a linear $C_1$-$C_4$-alkyl radical and Alk represents a linear $C_1$-$C_7$-alkylene radical, the free valencies of which originate from the two terminal carbon atoms.

12. A process according to any one of claims 1, 2 and 7–10, characterised in that the starting materials are so selected that a compound of formula I is manufactured in which in the radical of the partial formula II $R^1_a$ represents a linear $C_1$-$C_4$-alkyl radical and Alk represents a linear 1,1-alkylidene radical or a 1,1-alkylidene radical branched once.

13. A process according to any one of claims 1, 2 and 7–10, characterised in that the starting materials are so selected that a compound of formula I is manufactured in which the radical of the formula II is represented by the partial formula -CO-AAA-O-$R^1_a$ (IIA) in which $R^1_a$ represents a linear $C_1$-$C_4$-alkyl radical and AAA represents a radical of a common $\alpha$-amino acid limited by the general definition of Alk and in the form of an individual optical isomer or a mixture of several isomers.

14. A process according to any one of claims 1, 2 and 7–10, characterised in that the starting materials are so selected that a compound of formula I is manufactured in which in the radical of the partial formula II $R^1_a$ represents a linear $C_1$-$C_4$-alkyl radical and Alk represents methylene.

15. A process according to claim 1, characterised in that the starting materials are so selected that a compound of formula I is manufactured in which B represents a carbamoyl radical of the partial formula II in which $R^1_a$ represents $C_1$-$C_4$-alkyl and Alk represents $C_1$-$C_4$-alkylene and each of $A^1$, $A^2$ and $A^3$ represents hydrogen, alkanoyl having up to 10 carbon atoms, lower alkoxycarbonyl, 2-(2-methoxyethoxy)-ethoxycarbonyl, di-lower alkylaminocarbonyl or a carbamoyl radical of the partial formula II in which $R^1_a$ represents $C_1$-$C_4$-alkyl and Alk represents $C_1$-$C_4$-alkylene, $A^1$, $A^2$ and $A^3$ all having the same meaning.

16. A process according to claim 1, characterised in that the starting materials are so selected that a compound of formula I is manufactured in which B represents ethoxycarbonylmethylaminocarbonyl or 2-ethoxycarbonylethylaminocarbonyl and each of $A^1$, $A^2$ and $A^3$ represents ethoxycarbonylmethylaminocarbonyl, 2-ethoxy carbonylethylaminocarbonyl, hydrogen, n-octanoyl, ethoxycarbonyl, 2-(2-methoxyethoxy)-ethoxycarbonyl or diethylaminocarbonyl, $A^1$, $A^2$ and $A^3$ all having the same meaning.

17. A process according to claim 1, 15 or 16, characterised in that the starting materials are so selected that a compound of formula I is manufactured in which each of $A^1$, $A^2$ and $A^3$ represents ethoxycarbonylmethylaminocarbonyl, 2-ethoxycarbonylethylaminocarbonyl, noctanoyl, ethoxycarbonyl or 2-(2-methoxyethoxy)-ethoxycarbonyl, $A^1$, $A^2$ and $A^3$ all having the same meaning.

18. A process according to claim 1, 15 or 16, characterised in that the starting materials are so selected that a compound of formula I is manufactured in which each of $A^1$, $A^2$ and $A^3$ represents di-lower alkylaminocarbonyl.

19. A process according to claim 1 or 2, characterised in that the starting materials are so selected that N,O,O',O"-tetra-(ethoxycarbonylmethylcarbamoyl)-desferrioxamine B or N,O,O',O"-tetra-(2-ethoxycarbonylethylaminocarbonyl)-desferrioxamine B is manufactured.

20. A process according to claim 1 or 3, characterised in that the starting materials are so selected that N-(ethoxycarbonylmethylcarbamoyl)-O,O',O"-trioctanoyldesferrioxamine B or N-(2-ethoxycarbonylethylaminocarbonyl)-O,O',O"-trioctanoyl-desferrioxamine B is manufactured.

21. A process according to claim 1 or 3, characterised in that the starting materials are so selected that N-(ethoxycarbonylmethylcarbamoyl)-O,O',O"-triethoxycarbonyl-desferrioxamine B, N-(2-ethoxycarbonylethyl aminocarbonyl)-O,O',O"-tri-(ethoxycarbonyl)-desferrioxamine B or N-(2-ethoxycarbonylethylaminocarbonyl)O,O',O"-tri-(ethoxycarbonylmethylaminocarbonyl)-desferrioxamine B is manufactured.

22. A process according to claim 1 or 3, characterised in that the starting materials are so selected that N-(ethoxycarbonylmethylcarbamoyl)-O,O',O"-tri-[2-(2methoxyethoxy)-ethoxycarbonyl]-desferrioxamine B or N(2-ethoxycarbonylethylaminocarbonyl)-O,O',O"-tri-[2-(2methoxyethoxy)-ethoxycarbonyl-desferrioxamine B is manufactured.

23. A process according to claim 1 or 3, characterised in that the starting materials are so selected that N-ethoxycarbonylmethylaminocarbonyl-O,O',O"-tri-(diethylaminocarbonyl)-desferrioxamine B is manufactured.

**Revendications pour les Etats contractants: ES, GR**

1. Procédé pour la préparation d'un composé de formule I

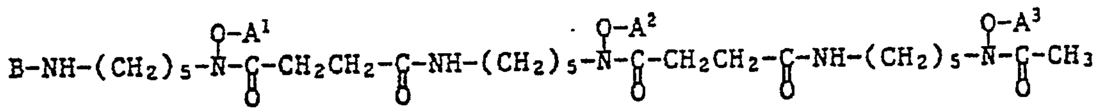

(I)

dans laquelle B représente un reste carbamoyle de formule partielle -CO-NH-Alk-CO-O-$R1_a$ (II) où $R1_a$ représente un alkyle en $C_1$-$C_4$ ou un alcényle en $C_2$-$C_4$ et Alk représente un alkylène en $C_1$-$C_7$ non substitué ou substitué par l'hydroxyle, un alcanoyloxy en $C_1$-$C_4$, un amino, un alcoxy en $C_1$-$C_4$ carbonyle, un carbamoyle, un phényle, l'hydroxyphényle, le méthoxyphényle ou l'indolyle et chacun des symboles $A^1$, $A^2$ et $A^3$ représentent indépendamment les uns des autres, l'hydrogène, un reste acyle Ac dérivé d'un acide carboxylique ou un reste carbamoyle de formule partielle II défini ci-dessus ou un sel d'un tel composé présentant des propriétés salifiables, caractérisé

a) en ce que l'on fait réagir un dérivé de la desferrioxamine B de formule IV

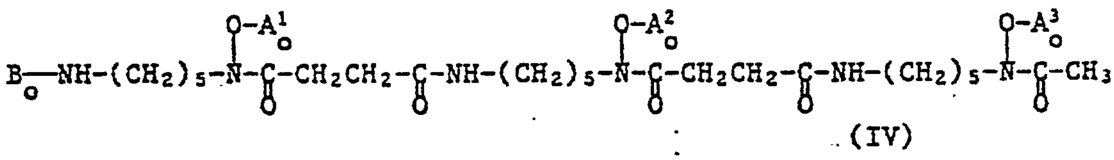

(IV)

où $B_O$ représente l'hydrogène ou un groupe silyle Sil organique et où chacun des symboles $A^1_O$, $A^2_O$ et $A^3_O$ représentent, indépendamment les uns des autres, l'hydrogène, un groupe silyle Sil organique ou un reste acyle Ac défini ci-dessus, avec un ester d'un acide isocyanatocarboxylique de formule III

$$O=C=N-Alk-CO-O-R^1_a \qquad (III)$$

où Alk et $R^1_a$ ont les significations données ci-dessus et dans lequel se trouve éventuellement présent dans le reste Alk un groupe amino supplémentaire sous forme protégée ou

b) en ce que l'on fait réagir un dérivé de la desferrioxamine B de formule V

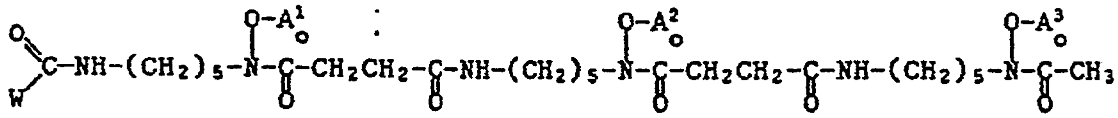

(V)

dans laquelle W représente le chlore ou le 1-imidazolyle et où chacun des symboles $A^1_O$, $A^2_O$ et $A^3_O$ représentent, indépendamment les uns des autres, l'hydrogène, un groupe silyle Sil organique ou un reste acyle Ac défini, ci-dessus, avec un ester de l'acide aminé de formule VI

$$H_2N\text{-}Alk\text{-}CO\text{-}O\text{-}R^1_a \qquad (VI)$$

où alk et $R1_a$ ont les significations données ci-dessus et dans lequel se trouve éventuellement présent dans le reste Alk un groupe amino supplémentaire sous forme protégée, et en ce que l'on clive les groupes silyles et/ou les groupes protecteurs de l'amino éventuellement présents en libérant les groupes hydroxyles ou amino correspondants et, si désiré, en ce que l'on acyle le composé de formule I obtenu où au moins l'un des symboles $A^1$, $A^2$ et $A^3$ est l'hydrogène, en un composé de formule I où les symboles correspondants représentent Ac ou un reste carbamoyle de formule partielle II et/ou, si désiré, en ce que l'on transforme le composé libre de formule I obtenu ayant des propriétés salifiables en un sel et/ou en ce qu'on libère un composé de formule I d'un tel sel.

2. Procédé pour la préparation d'un composé de formule I selon la revendication 1, dans laquelle chacun des symboles $A^1$, $A^2$, $A^3$ et B représente un reste de formule partielle -CO-NH-Alk-CO-O-$R^1_a$ (II) où $R^1a$ représente un alkyle en $C_1$-$C_4$ et Alk représente un alkylène en $C_1$-$C_7$ non substitué ou substitué par l'hydroxyle, un alcanoyloxy en $C_1$-$C_4$, l'amino, un alcoxycarbonyle en $C_1$-$C_4$ un carbamoyle, un phényle, l'hydroxyphényle, le méthoxyphényle ou l'indolyle ou d'un sel d'un tel composé présentant des propriétés salifiables, caractérisé en ce que l'on fait réagir la desferrioxamine B ou l'un de ses sels d'addition d'acide avec un ester d'un acide isocyanatocarboxylique de formule

$$O{=}C{=}N\text{-}Alk\text{-}CO\text{-}O\text{-}R^1_a \qquad (III)$$

où Alk et $R1_a$ ont les significations données ci-dessus et dans lequel se trouve éventuellement présent dans le reste Alk un groupe amino sous forme protégée et ce qu'on libère un groupe amino éventuellement présent par clivage du groupe protecteur de l'amino et, si désiré, en ce que l'on transforme le composé libre de formule I présentant des propriétés salifiables en un sel et/ou en ce qu'on libère un composé de formule I d'un tel sel.

3. Procédé selon la revendication 1, caractérisé en ce que, pour la préparation des composés de formule I où B a une autre signification que $A^1$ $A^2$ et $A^3$, on part d'un composé de formule IV où $B_O$ ainsi qu'au moins l'un des symboles $A^1_O$, $A^2_O$ et $A^3_O$ représentent un groupe silyle organique de formule

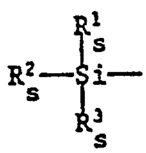

(Sil),

dans laquelle $R^1_s$ et $R^2_s$ représentent, indépendamment l'un de l'autre, un hydrocarbyle en $C_1$-$C_8$ non substitué et $R^3_s$ représente un hydrocarbyle en $C_1$-$C_8$ non substitué ou le chlore.

4. Procédé selon la revendication 1, caractérisé en ce que, pour la préparation des composés de formule I où $A^1$, $A^2$ et $A^3$ représentent l'hydrogène, on part d'un composé de formule IV où $B_O$, $A^1$, $A^2$ et $A^3_O$ représentent tous le groupe Sil défini dans la revendication 3.

5. Procédé selon la revendication 3 ou 4, caractérisé en ce que l'on fait réagir le produit de départ de formule IV défini dans ces revendications, avec le réactif de formule III défini dans la revendication 1 et en ce que l'on clive ensuite les groupes silyles par solvolyse.

6. Procédé selon la revendication 5, caractérisé en ce que l'on prépare le produit de départ de formule IV in situ, en faisant réagir la desferrioxamine B ou l' un de ses sels avec un réactif de silylation de formule Sil-Hal où Hal représente le brome ou le chlore et Sil a la signification indiquée dans la revendication 3, dans une base hétérocyclique aprotique du type pyridine et en ce que l'on procède ensuite dans

le même milieu réactionnel à la réaction avec le réactif de formule III et au clivage des groupes silyles.

7. Procédé selon la revendication 1, caractérisé en ce que l'on choisit les produits de départ de telle façon que l ' on prépare un composé de formule I où $A^1$, $A^2$ et $A^3$ ont la même signification.

8. Procédé selon la revendication 1, caractérisé en ce que l'on choisit les produits de départ de telle façon que l' on prépare un composé de formule I où $A^1$, $A^2$ et $A^3$ représentent l' hydrogène.

9. Procedé selon la revendication 1, caractérisé en ce que l'on choisit les produits de départ de telle façon que l' on prépare un composé de formule I où $A^1$, $A^2$ et $A^3$ représentent chacun un seul et même reste acyle Ac ou un reste carbamoyle de formule partielle II.

10. Procédé selon la revendication 1, caractérisé en ce que l'on choisit les produits de départ de telle façon que l'on prépare un composé de formule I dans laquelle chacun des symboles $A^1$, $A^2$ et $A^3$ représente un reste de formule partielle -CO-NH-Alk-CO-O-$R^1_a$ (II) où $R_a$ représente un alkyle en $C_1$-$C_4$ et Alk représente un alkylène en $C_1$-$C_7$ non substitué ou substitué par l'hydroxyle, un alcanoyloxy en $C_1$-$C_4$, l'amino, un alcoxycarbonyle en $C_1$-$C_4$, un carbamoyle, un phényle, l'hydroxyphényle, le méthoxyphényle ou l'indolyle ou un sel d'un tel composé présentant des propriétés salifiables.

11. Procédé selon la revendication 1, caractérisé en ce que l'on choisit les produits de départ de telle façon que l'on prépare un composé de formule I où dans le reste de formule partielle II $R^1_a$ représente un reste alkyle en $C_1$-$C_4$ linéaire et Alk représente un reste alkylène en $C_1$-$C_7$ linéaire dont les valences libres partent des deux atomes de C terminaux.

12. Procédé selon l'une des revendications 1, 2 et 7 à 10, caractérisé en ce que l'on choisit les produits de départ de telle façon que l'on prépare un composé de formule I où dans le reste de formule partielle II $R^1_a$ représente un alkyle en $C_1$-$C_4$ linéaire et Alk représente un 1,1-alkylidène linéaire ramifié une fois.

13. Procédé selon l'une des revendications 1, 2 et 7 à 10, caractérisé en ce que l'on choisit les produits de départ de telle façon que l'on prépare un composé de formule I où le reste de formule II est représenté par la formule partielle -CO-AAA-O-$R^1_a$ (IIA) où $R^1_a$ représente un alkyle en $C_1$-$C_4$ linéaire et AAA représente un reste limité par la définition générale de Alk d'un $\alpha$-amino-acide courant sous forme d'un isomère optique individuel ou d'un mélange de plusieurs isomères.

14. Procédé selon l'une des revendications 1, 2 et 7 à 10, caractérisé en ce que l'on choisit les produits de départ de telle façon que l'on prépare un composé de formule I où dans le reste de formule partielle II $R^1_a$ représente un alkyle en $C_1$-$C_4$ linéaire et Alk représente le méthylène.

15. Procédé selon la revendication 1, caractérisé en ce que l'on choisit les produits de départ de telle façon que l'on prépare un composé de formule I dans laquelle B représente un reste carbamoyle de formule partielle II où $R^1_a$ représente un alkyle en $C_1$-$C_4$ et Alk représente un alkylène en $C_1$-$C_4$ et $A^1$, $A^2$ et $A^3$ représentent l'hydrogène, un reste carbamoyle de formule partielle II où $R^1_a$ représente un alkyle en $C_1$-$C_4$ et Alk représente un alkylène en $C_1$-$C_4$ un alcanoyle ayant jusqu'à 10 atomes de C, un alcoxy inférieur carbonyle, le 2-(2-méthoxy-éthoxy)-éthoxy-carbonyle ou un dialkyle inférieur amino-carbonyle, $A^1$, $A^2$ et $A^3$ ayant la même signification.

16. Procédé selon la revendication 1, caractérisé en ce que l'on choisit les produits de départ de telle façon que l'on prépare un composé de formule I dans laquelle B représente un éthoxycarbonylméthylaminocarbonyle ou le 2-éthoxycarbonyléthylaminocarbonyle et $A^1$, $A^2$ et $A^3$ représentent un éthoxycarbonylméthylaminocarbonyle, le 2-éthoxycarbonyléthylaminocarbonyle, l'hydrogène, le n-octanoyle, l'éthoxycarbonyle, le 2(2-méthoxy-éthoxy)-éthoxycarbonyle ou le diéthylaminocarbonyle, $A^1$ $A^2$ et $A^3$ ayant la même signification.

17. Procédé selon la revendication 1, 15 ou 16, caractérisé en ce que l'on choisit les produits de départ de telle façon que l'on prépare un composé de formule I où $A^1$, $A^2$ et $A^3$ représentent un éthoxycarbonylméthylaminocarbonyle, le 2-éthoxycarbonyléthylaminocarbonyle, le n-octanoyle, un éthoxycarbonyle ou le 2-(2-méthoxyéthoxy)-éthoxycarbonyle, $A^1$, $A^2$ et $A^3$ ayant la même signification.

18. Procédé selon la revendication 1, 15 ou 16, caractérisé en ce que l'on choisit les produits de départ de telle façon que l'on prépare un composé de formule I où $A^1$, $A^2$ et $A^3$ représentent un dialkyle inférieur aminocarbonyle.

19. Procédé selon la revendication 1 ou 2, caractérisé en ce que l'on choisit les produits de départ de telle façon que l'on prépare la N,O,O',O"-tétra(éthoxycarbonylméthylcarbamoyl)-desferrioxamine B ou la N, O, O', O"-tétra-(2-éthoxycarbonyl-éthylamino-carbonyl)desferrioxamine B.

20. Procédé selon la revendication 1 ou 3, caractérisé en ce que l'on choisit les produits de départ de telle façon que l'on prépare la N-(éthoxycarbonylméthylcarbamoyl)-O,O',O"-tri-octanoyl-desferrioxamine B ou la N-(2-éthoxycarbonyl-éthylamino-carbonyl)-O,O',O"trioctanoyl-desferrioxamine B.

21. Procédé selon la revendication 1 ou 3, caractérisé en ce que l'on choisit les produits de départ de telle façon que l'on prépare la N-(éthoxycarbonylméthylcarbamoyl)-O,O',O"-tri-éthoxycarbonyl-desferrioxamine B, la N-(2-éthoxycarbonyl-éthylamino-carbonyl)O,O',O"-tri-(éthoxycarbonyl)-desferrioxamine B ou la N-(2-éthoxycarbonyl-éthylamino-carbonyl)-O,O',O"-tri(éthoxycarbonylméthylaminocarbonyl)-desferrioxamine B.

22. Procédé selon la revendication 1 ou 3, caractérisé en ce que l'on choisit les produits de départ de telle façon que l'on prépare la N-(éthoxycarbonylméthylcarbamoyl)-O,O',O-tri-[2-(2-méthoxy-éthoxy)éthoxycarbonyl]-desferrioxamine B ou la N-(2-éthoxycarbonyl-éthylamino-carbonyl)-O,O',O"-tri-[2-(2-méthoxyéthoxy)-éthoxy-carbonyl]-desferrioxamine B.

23. Procédé selon la revendication 1 ou 3, caractérisé en ce que l'on choisit les produits de départ de telle façon que l'on prépare la N-éthoxycarbonylméthylaminocarbonyl-O,O',O"-tri-(diéthylaminocarbonyl)-desferrioxamine B.

**Revendications pour les Etats contractants: AT, BE, CH, DE, FR, GB, IT, LI, LU, NL, SE**

1. Composé de formule I

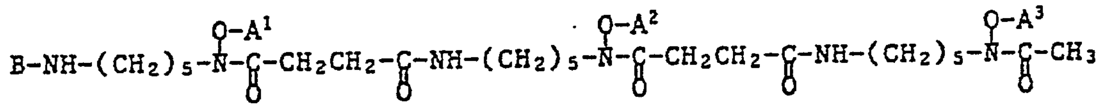

(I)

dans laquelle B représente un reste carbamoyle de formule partielle -CO-NH-Alk-CO-O-$R^1_a$ (II) où $R^1_a$ représente un alkyle en $C_1$-$C_4$ ou un alcényle en $C_2$-$C_4$ et Alk représente un alkylène en $C_1$-$C_7$ non substitué ou substitué par l'hydroxyle, un alcanoyloxy en $C_1$-$C_4$, un amino, un alcoxy en $C_1$-$C_4$ carbonyle, un carbamoyle, un phényle, l'hydroxyphényle, le méthoxyphényle ou l'indolyle et chacun des symboles $A^1$, $A^2$ et $A^3$ représentent, indépendamment les uns des autres, l'hydrogène, un reste acyle Ac dérivé d'un acide carboxylique ou un reste carbamoyle de formule partielle II défini ci-dessus ou un sel d'un tel composé présentant des propriétés salifiables.

2. Composé selon la revendication 1, où $A^1$, $A^2$ et $A^3$ ont la même signification.

3. Composé selon la revendication 1, où $A^1$, $A^2$ et $A^3$ représentent l'hydrogène.

4. Composé selon la revendication 1, où $A^1$, $A^2$ et $A^3$ représentent un seul et même reste acyle Ac ou un reste carbamoyle de formule partielle II.

5. Composé selon la revendication 2 de formule I, dans laquelle chacun des symboles $A^1$, $A^2$, $A^3$ et B représente un reste de formule partielle -CO-NH-Alk-CO-O-$R^1_a$ (II) où $R^1_a$ représente un alkyle en $C_1$-$C_4$ et Alk représente un alkylène en $C_1$-$C_7$ non substitué ou substitué par l'hydroxyle, un alcanoyloxy en $C_1$-$C_4$, l'amino, un alcoxycarbonyle en $C_1$-$C_4$, un carbamoyle, un phényle, l'hydroxyphényle, le méthoxyphényle ou l'indolyle ou un sel d'un tel composé présentant des propriétés salifiables.

6. Composé selon l'une des revendications 1 à 5, où dans le reste de formule partielle II $R^1_a$ représente un reste alkyle en $C_1$-$C_4$ linéaire et Alk représente un reste alkylène en $C_1$-$C_7$ linéaire dont les valences libres partent des deux atomes de C terminaux.

7. Composé selon l'une des revendications 1 à 5, où dans le reste de formule partielle II $R^1_a$ représente un alkyle en $C_1$-$C_4$ linéaire et Alk représente un 1,1-alkylidène linéaire ramifié une fois.

8. Composé selon l'une des revendications 1 à 5, où le reste de formule II est représenté par la formule partielle -CO-AAA-O-$R^1_a$ (IIA) où $R^1_a$ représente un alkyle en $C_1$-$C_4$ linéaire et AAA représente un reste limité par la définition générale de Alk d'un $\alpha$-amino-acide courant sous forme d'un isomère optique individuel ou d'un mélange de plusieurs isomères.

9. Composé selon l'une des revendications 1 à 5, où dans le reste de formule partielle II $R^1_a$ représente un alkyle en $C_1$-$C_4$ linéaire et Alk représente le méthylène.

10. Composé selon la revendication 1, dans laquelle B représente un reste carbamoyle de formule partielle II où $R^1_a$ représente un alkyle en $C_1$-$C_4$ et Alk représente un alkylène en $C_1$-$C_4$, et $A^1$, $A^2$ et $A^3$ représentent l'hydrogène, un reste carbamoyle de formule partielle II où $R^1_a$ représente un alkyle en $C_1$-$C_4$ et Alk représente un alkylène en $C_1$-$C_4$, un alcanoyle ayant jusqu'à 10 atomes de C, un alcoxy inférieur carbonyle, le 2-(2-méthoxy-éthoxy)-éthoxy-carbonyle ou un dialkyle inférieur amino-carbonyle, $A^1$, $A^2$ et $A^3$ ayant la même signification.

11. Composé selon la revendication 1, dans lequel B représente un éthoxycarbonylméthylaminocarbonyle ou le 2-éthoxycarbonyléthylaminocarbonyle et $A^1$, $A^2$ et $A^3$ représentent un éthoxycarbonylméthylaminocarbonyle, le 2-éthoxycarbonyléthylaminocarbonyle, l'hydrogène, le n-octanoyle, l'éthoxycarbonyle, le 2-(2-méthoxy-éthoxy)éthoxycarbonyle ou le diéthylaminocarbonyle, $A^1$, $A^2$ et $A^3$ ayant la même signification.

12. Composé selon la revendication 1, 10 ou 11, où $A^1$, $A^2$ et $A^3$ représentent un éthoxycarbonylméthylaminocarbonyle, le 2-éthoxycarbonyléthylaminocarbonyle, le n-octanoyle, un éthoxycarbonyle ou le 2-(2-méthoxyéthoxy)-éthoxycarbonyle $A^1$, $A^2$ et $A^3$ ayant la même signification.

13. Composé selon la revendication 1 ou 10, où $A^1$, $A^2$ et $A^3$ représentent un dialkyle inférieur aminocarbonyle.

14. N,O,O',O"-tétra-(éthoxycarbonylméthylcarbamoyl)-desferrioxamine B et N,O,O',O"-tétra-(2-éthoxycarbonyl-éthylamino-carbonyl)-desferrioxamine B selon la revendication 1.

15. N-(éthoxycarbonylméthylcarbamoyl)-O,O',O"tri-octanoyl-desferrioxamine B et N-(2-éthoxycarbonylethylamino-carbonyl)-O,O',O"-trioctanoyl-desferrioxamine B selon la revendication 1.

16. N-(éthoxycarbonylméthylcarbamoyl)-O,O',O"-tri-éthoxycarbonyl-desferrioxamine B, N-(2-éthoxycarbonyléthylamino-carbonyl)-O,O',O"-tri-(éthoxycarbonyl)-desferrioxamine B et -(2-éthoxycarbonyléthylamino-carbonyl)0, 0', 0"-tri-(éthoxycarbonylméthylaminocarbonyl)-desferrioxamine B selon la revendication 1.

17. N-(éthoxycarbonylméthylcarbamoyl)-O,O',O"-tri-[2-(2-méthoxy-éthoxy)-éthoxycarbonyl]-desferrioxamine B et N-(2-éthoxycarbonyl-éthylamino-carbonyl)-O,O',O"tri-[2-(2-méthoxy-éthoxy)-éthoxycarbonyl]-desferrioxamine B selon la revendication 1.

18. N-éthoxycarbonylméthylaminocarbonyl-O,O',O"-tri-(diéthylamino-carbonyl)-desferrioxamine B selon la revendication 1.

19. Procédé pour la préparation d'un composé de formule I selon la revendication 1, caractérisé

a) en ce que l'on fait réagir un dérivé de la desferrioxamine B de formule IV

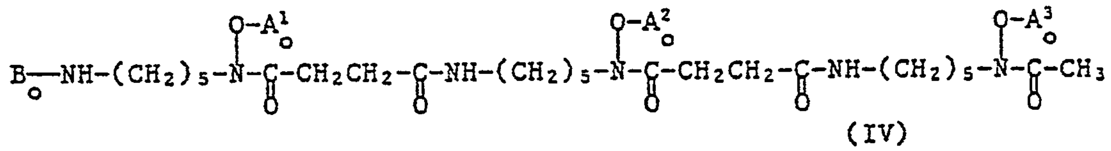

(IV)

où $B_o$ représente l'hydrogène ou un groupe silyle Sil organique et où chacun des symboles $A^1_O$, $A^2_O$ et $A^3_O$ représentent, indépendamment les uns des autres, l'hydrogène, un groupe silyle Sil organique ou un reste acyle Ac défini ci-dessus, avec un ester d'un acide isocyanatocarboxylique de formule III

$$O{=}C{=}N{-}Alk{-}CO{-}O{-}R^1_a \qquad \text{(III)}$$

où Alk et $R^1_a$ ont les significations données ci-dessus et dans lequel se trouve éventuellement présent dans le reste Alk un groupe amino supplémentaire sous forme protégée ou

b) en ce que l'on fait réagir un dérivé de la desferrioxamine B de formule V

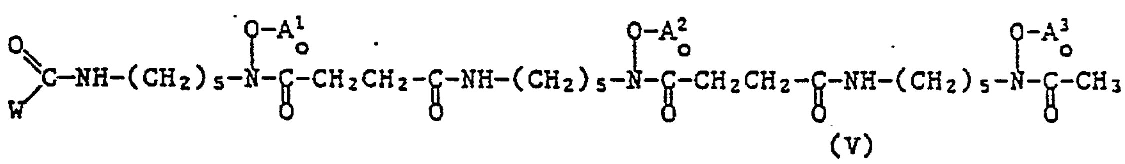

(V)

dans laquelle W représente le chlore ou le 1-imidazolyle et où chacun des symboles $A^1_a$, $A^2_O$ et $A^3_O$ représentent, indépendamment les uns des autres, l'hydrogène, un groupe silyle Sil organique ou un reste acyle Ac défini, ci-dessus, avec un ester de l'acide aminé de formule VI

$$H_2N{-}Alk{-}CO{-}O{-}R^1_a \qquad \text{(VI)}$$

où Alk et $R^1_a$ ont les significations données ci-dessus et dans lequel se trouve éventuellement présent dans le reste Alk un groupe amino supplémentaire sous forme protégée, et en ce que l'on clive les groupes silyles et/ou les groupes protecteurs de l'amino éventuellement présents en libérant les groupes hydroxyles ou amino correspondants et, si désiré, en ce que l'on acyle le composé de formule I obtenu où au moins l'un des symboles $A^1$, $A^2$ et $A^3$ est l'hydrogène, en un composé de formule I où les symboles correspondants représentent Ac ou un reste carbamoyle de formule partielle II et/ou, si désiré, en ce que l'on transforme le composé libre de formule I obtenu ayant des propriétés salifiables en un sel et/ou en libérant un composé de formule I d'un tel sel.

20. Composé selon l'une des revendications 1 à 18, destiné à être utilisé dans un procédé pour le traitement thérapeutique du corps humain ou animal.

21. Compositions pharmaceutiques contenant, à côté d'un support pharmaceutique, comme substance active, un composé de formule I selon l'une des revendications 1 à 18 ou un sel pharmaceutiquement utilisable d'un tel composé ayant des propriétés salifiables.

22. Compositions pharmaceutiques selon la revendication 21 sous forme de dragées, de comprimés ou de capsules destinés à l'administration orale.

23. Utilisation d'un composé selon l'une des revendications 1 à 18 pour la préparation de compositions pharmaceutiques destinées à être utilisées pour le traitement des états pathologiques ches les êtres à sang chaud y compris l'homme, liés à un excès en fer (III) ou en aluminium dans le corps ou provoqués par des organismes pathogènes dépendant du fer (III).